# EUROPÄISCHE PATENTSCHRIFT

(11) **EP 3 359 495 B1**
(45) Veröffentlichungstag und Bekanntmachung des Hinweises auf die Patenterteilung: **30.10.2019**
(21) Anmeldenummer: 16775095.9
(22) Anmeldetag: 15.09.2016
(51) Int. Cl.: C03B 23/045, A61M 5/28, C03B 23/09, C03B 23/18, A61M 5/31

(54) **VORRICHTUNG UND VERFAHREN ZUR HERSTELLUNG EINES MEDIZINISCHEN GLASBEHÄLTERS**
DEVICE AND METHOD FOR PRODUCING A MEDICAL GLASS CONTAINER
DISPOSITIF ET PROCÉDÉ DE FABRICATION D'UN RÉCIPIENT MÉDICAL EN VERRE

(30) Priorität: 08.10.2015 DE 102015117215
(43) Veröffentlichungstag der Anmeldung: 15.08.2018
(73) Patentinhaber: Gerresheimer Bünde GmbH, 32257 Bünde (DE)
(72) Erfinder: SCHNELLE, Dirk, 32257 Bünde (DE); WITTLAND, Frank, 32130 Enger (DE)
(74) Vertreter: Hannke, Christian
(86) Internationale Anmeldenummer: PCT/EP2016/071760
(87) Internationale Veröffentlichungsnummer: WO 2017/060052

(56) Entgegenhaltungen:
- WO-A1-2012/085619
- WO-A2-2006/039705
- US-A1- 2004 231 361
- US-A1- 2012 060 558
- US-A1- 2015 114 043
- US-B1- 6 415 631

## Beschreibung

Die Erfindung betrifft eine Vorrichtung zur Herstellung eines hochreinen medizinischen Glasbehälters aus einem sich entlang einer axialen Richtung (X) erstreckenden hohlzylindrischen Glasrohling mit zumindest einem offenen Ende, wobei der Glasrohling ausgehend von dem offenen Ende ein sich in axialer Richtung (X) erstreckenden formbaren Formabschnitt aufweist, wobei die Vorrichtung umfasst:
a. ein erstes Formwerkzeug, welches einen Formstift aufweist, wobei der Formstift durch das offene Ende des hohlzylindrischen Glasrohlings in dessen Formabschnitt entlang der axialen Richtung (X) verlagerbar ist, wobei der Formstift in einer Fixiereinrichtung des ersten Formwerkzeugs fixiert ist;
b. ein zweites Formwerkzeug, durch welches zumindest der Formabschnitt des hohlzylindrischen Glasrohlings verformbar ist, wobei der Formabschnitt durch das zweite Formwerkzeug derart verformbar ist, dass eine innere Oberfläche des Formabschnitts an dem Formstift anliegt, wodurch der Formabschnitt einen Kanal ausbildet;

Gattungsgemäße medizinische Glasbehälter sind insbesondere Spritzen aber auch Ampullen oder Karpulen. Bei der Neuentwicklung von Medikamenten wird der erste zu untersuchende Medikamentenansatz in Glasampullen, sogenannten Vials gelagert. Dabei werden die ersten Stabilitätsuntersuchungen in diesen Vials durchgeführt. Ein Glasbehälter, der aus pharmazeutischer Sicht die gleichen oder sehr angenäherte Eigenschaften wie das ursprünglich verwendete Vial hat, vermeidet unerwünschte Eigenschaftsänderungen bei der Lagerung. Derartige Glasbehälter sind in der Pharmabranche als Behältnisse für sensible/ reaktive Formulierungen wünschenswert, da packmittelbedingte unerwünschte Eigenschaftsänderungen entfallen.

Die Herstellung vorfüllbarer Ganzglasspritzen erfolgt in der Regel durch Erzeugen von Abschnitten von Glasröhren (Glasrohlingen), die als Halbzeug dienen, und nachfolgender Umformung bei hohen Temperaturen. Bei der Umformung werden Formwerkzeuge eingesetzt, die eine ausreichende Wärmeformbeständigkeit und Verschleißfestigkeit bei gleichzeitig hoher Duktilität aufweisen. Die Glasspritzen werden in der Regel aus sehr chemikalien- und temperaturbeständigen Gläsern wie Borosilikatglas oder Quarzglas hergestellt. Dabei ist insbesondere die Ausformung des Spritzenkonus kritisch. Der Spritzenkonus weist einen Kanal auf, welcher die Kanüle mit dem Hohlraum der Spritze, in dem das zu verabreichende Medium aufbewahrt wird, verbindet,.

Der Spritzenkonus kann unter anderem als ein sogenannter Luer-Konus ausgebildet werden. Ein Luer-Ansatz-, Luer-Steck- oder Luer-Slip-System ist ein genormtes (ISO-Norm 594) Verbindungssystem für Schlauchsysteme im medizinischen Bereich und garantiert die Kompatibilität zwischen verschiedenen Herstellern. Es findet unter anderem Anwendung bei Spritzen, Kanülen und Infusions-Schläuchen. Ein männlicher Luer-Konus ist ein flacher (Steigung 6%) Hohlkegel mit relativ großer Oberfläche und daher guter Haftung mit einem aufgesteckten (weiblichen) Luer-Ansatz von beispielsweise einer Kanüle. Durch die kegelförmige Konstruktion beider Verbindungsteile wird eine ausreichende Dichtung erreicht.

Ferner werden auch Glasspritzen mit sogenannten Staked-in Nadelspitzen (SIN) verwendet. Hier wird die Kanüle in dem Kanal üblicherweise durch eine Klebeverbindung befestigt. In der Regel ist es notwendig, dass der Kanal der SIN-Spritzen einen kleineren Durchmesser als bei Spritzen mit einem Luer-Konus aufweist, um das Einkleben der Kanüle zu erleichtern.

Die Herstellung des Spritzenkonus erfolgt durch abschnittsweises Aufheizen des Glasrohlings. Ist der Glasrohling in einem formbaren Zustand, wird die äußere Formgebung mittels eines zweiten Formwerkzeugs und die innere Formgebung durch Gegenhalten eines sogenannten Formstifts oder Formdorns, welcher ein Bestandteil eines ersten Formwerkzeugs ist, hergestellt.

Während die Formwerkzeuge auf der Außenseite der Röhre unkritisch sind, da ein eventuell entstehender Abrieb nicht mit dem Füllgut in Kontakt kommt, sind alle Werkzeuge, welche innerhalb des Glasrohlings Anwendung finden, kritisch zu betrachten. Die derzeit im Stand der Technik eingesetzten Werkzeuge bestehen nahezu zu 100% aus Wolfram. Dieses Material eignet sich zwar gut für die außen anliegenden Werkzeuge, für Werkzeuge, welche innerhalb des Glasbehältnisses eingesetzt werden, ist es allerdings weniger geeignet. Der Formstift für den Kanal des Spritzenkonus ist sehr klein, und heizt sich durch die geringe Wärmekapazität stark auf, so dass Oxide entstehen, die zu Verschleiß und Abrieb führen. Der Abrieb des Formstifts liegt in einer Größenordnung, die es erforderlich macht, dass der Formstift in der Regel nach ca. 1 Stunde Betrieb ausgetauscht werden muss. Dieser Abrieb schlägt sich zumindest anteilig in dem Kanal nieder. Dort kann er später durch das Füllgut absorbiert werden, und eventuell zur Beeinträchtigung von dessen Wirksamkeit und/oder Verträglichkeit führen. Gelöste Wolfram-Polyanione in vorgefüllten Spritzen interagieren mit einigen Proteinen, so dass diese aggregieren. Partikelbildung und eine Änderung der Wirksamkeit des Arzneimittels oder körpereigener Proteine sind die Folge. Insbesondere neuartige biotechnologische Wirkstoffe weisen oft langkettige Molekülketten auf, welche sehr empfindlich hinsichtlich einer Wechselwirkung mit Spurenelementen ist. Diese Produkte können somit nicht in normalen Spritzen gelagert werden. Durch ein Extraktionsverfahren und nachfolgender analytischer Bestimmung sind die Rückstände durch den Abrieb nachweisbar. Ausgehend von Unverträglichkeitsvorfällen in der Industrie hat bereits eine Verständigung auf zulässige Grenzwerte stattgefunden. Demnach werden nach derzeitigem Stand Spritzen mit einem Wolfram-Gehalt von < 50 ng als hochreine Spritzen bezeichnet. Standard-Spritzen können dagegen Werte zwischen 500 und 2500 ng aufweisen. In der Industrie werden derzeit Lösungen durch Waschprozesse oder Ersatz-Metalle wie Platin bereitgestellt. Diese können den Gehalt aber nur reduzieren, bzw. bringen ein Ersatzmetall ein, welches seinerseits auch nicht frei von Wechselwirkungen sein muss.

Alternative Materialien für solche Formstifte müssen eine hohe Temperaturbeständigkeit und eine hohe Temperaturwechsel-Beständigkeit (Thermoschockbeständigkeit) aufweisen. Ferner soll das Material geringe Neigung dazu haben, an dem Glasrohling während der Formung zu kleben. Viele der infrage kommenden Materialen weisen eine deutlich höhere Sprödigkeit im Vergleich zu metallischen Materialien auf. Bisher wurden die Formstifte in einer Fixiereinrichtung fixiert. Der Formstift wird dabei in einer Bohrung der Fixiereinrichtung angeordnet. Ferner wird der Formstift durch eine Fixierschraube, welche punktuellen Kontakt mit dem Formstift hat, fixiert. Durch einen derartigen punktuellen Kontakt können Formstifte mit einer erhöhten Sprödigkeit leicht brechen.

WO2012085619 A1 lehrt eine Vorrichtung zur Herstellung eines hochreinen medizinischen Glasbehälters aus einem hohlen Glaszylinder einschließlich einem äußeren Formwerkzeug und einem Stift als innerem Formwerkzeug, welcher in einer Haltevorrichtung fixiert ist. Die Haltevorrichtung wird in WO2012085619 A1 nicht definiert. Der Stift besteht im Inneren vorzugsweise aus Wolfram, und ist mit einer keramischen Beschichtung versehen, welche vorzugsweise aus Siliziumkarbid oder Bornitrid besteht.

Aufgabe der vorliegenden Erfindung ist es demnach, eine Vorrichtung beziehungsweise ein Verfahren zur Herstellung hochreiner Spritzen bereitzustellen, welche keinen beziehungsweise einen geringen Wolframgehalt aufweisen, wobei die Vorrichtung die Verwendung von Formstiften mit hoher Sprödigkeit erlaubt.

Diese Aufgabe wird in einem Aspekt gelöst durch eine Vorrichtung zur Herstellung eines hochreinen medizinischen Glasbehälters aus einem sich entlang einer axialen Richtung (X) erstreckenden hohlzylindrischen Glasrohling mit zumindest einem offenen Ende, wobei der Glasrohling ausgehend von dem offenen Ende ein sich in axialer Richtung (X) erstreckenden formbaren Formabschnitt aufweist, wobei die Vorrichtung umfasst:
a. ein erstes Formwerkzeug, welches einen Formstift aufweist, wobei der Formstift durch das offene Ende des hohlzylindrischen Glasrohlings in dessen Formabschnitt entlang der axialen Richtung (X) verlagerbar ist, wobei der Formstift in einer Fixiereinrichtung des ersten Formwerkzeugs fixiert ist;
b. ein zweites Formwerkzeug, durch welches zumindest der Formabschnitt des hohlzylindrischen Glasrohlings verformbar ist, wobei der Formabschnitt durch das zweite Formwerkzeug derart verformbar ist, dass eine innere Oberfläche des Formabschnitts an dem Formstift anliegt, wodurch der Formabschnitt einen Kanal ausbildet;

Die Vorrichtung zeichnet sich dadurch aus, dass die Fixiereinrichtung zumindest zwei backenartige Elemente aufweist, welche an den Formstift flächig anpressbar sind, wodurch der Formstift kraftschlüssig fixierbar ist.

Durch die erfindungsgemäße Ausführung der Fixiereinrichtung wird somit eine punktuelle Fixierung des Formstifts vermieden. Der Formstift wird nun zwischen zwei backenartigen Elementen gespannt und liegt dabei abschnittsweise flächig an diesen Elementen an, so dass eine kraftschlüssige Fixierung erfolgt. In einer derartigen Fixiereinrichtung können auch Formstifte mit einem geringen Durchmesser und hoher Sprödigkeit fixiert werden, ohne dass diese durch die Fixierung beschädigt werden oder brechen.

Vorzugsweise sind die backenartigen Elemente der Fixiereinrichtung einstückig ausgebildet.

Nach einem bevorzugten Gedanken der Erfindung erstreckt die Fixiereinrichtung sich entlang einer axialen Richtung (X') und umfasst einen ersten Abschnitt, einen zweiten Abschnitt und dritten Abschnitt. Vorzugsweise sind der zweite und der dritte Abschnitt als Kreiszylinder ausgebildet. Bevorzugt weist die Fixiereinrichtung eine erste Durchgangsbohrung auf, welche sich entlang einer axialen Mittelachse der Fixiereinrichtung erstreckt und in welcher der Formstift zumindest abschnittsweise anordenbar ist. Der Formstift ist dabei derart anordenbar, dass dieser über den ersten Abschnitt hinausragt. Dieser hinausragende Abschnitt wird bei der Verformung des Formabschnitts des Glasrohlings durch dessen offenes Ende in den Formabschnitt verlagert. Der Formabschnitt wird dann durch das zweite Formwerkzeug verformt so dass eine innere Oberfläche des Formabschnitts an dem in dem Formabschnitt befindlichem Abschnitt des Formstifts anliegt, wodurch der Formabschnitt des Glasrohlings einen Kanal ausbildet.

Gemäß einem weiteren bevorzugten Gedanken der Erfindung weist der zweite Abschnitt eine erste und eine zweite Schlitzung auf. Die backenartigen Elemente sind dabei durch diese erste und die zweite Schlitzung beabstandet. Vorzugsweise ist die erste Schlitzung in einer ersten Ebene angeordnet, welche durch einen ersten Vektor in axialer Richtung (X') und einen zweiten Vektor in radialer Richtung (Y') aufgespannt wird. Die zweite Schlitzung ist bevorzugt in einer zweiten Ebene angeordnet, welche durch den zweiten Vektor und einen dritten Vektor aufgespannt wird. Der dritte Vektor schließt mit der ersten Ebene einen Winkel α ein. Dieser Winkel α liegt vorzugsweise in einem Bereich zwischen 45° und 125° und besonders bevorzugt bei 90°. Ferner weisen die erste und die zweite Schlitzung eine gemeinsame Schnittgerade auf, welche sich entlang der radialer Richtung (Y') erstreckt.

Vorzugsweise weist der zweiten Abschnitt entlang der axialen Richtung (X') eine erste Länge auf, wobei die erste Schlitzung entlang der axialen Richtung (X') eine zweite Länge aufweist, welche kleiner als die erste Länge ist.

Nach einer weiteren bevorzugten Ausführungsform weist die Fixiereinrichtung in dem zweiten Abschnitt eine zweite Durchgangsbohrung auf welche sich in der radialen Richtung (Y') erstreckt, wobei die zweite Durchgangsbohrung zu der ersten Schlitzung offen ist und in axialer Richtung zwischen dem ersten Abschnitt der Fixiereinrichtung und der ersten Schlitzung angeordnet ist.

Vorzugsweise weist der zweite Abschnitt eine dritte Durchgangsbohrung auf, deren Mittelachse senkrecht auf der ersten Ebene steht und hinsichtlich der axialen Mittelachse der Fixiereinrichtung radial außermittig angeordnet ist. Bevorzugt ist in dieser dritten Durchgangsbohrung ein Befestigungsmittel anbringbar, durch welches eine Schlitzweite der ersten Schlitzung reduzierbar ist, wodurch die backenartigen Elemente an dem Formstift anpressbar sind. Ein solches Befestigungsmittel könnte beispielsweise eine Spannschraube sein, durch welche eine Kraft auf die beiden backenartigen Elemente beaufschlagt wird, so dass diese zusammengedrückt werden, wodurch der Formstift durch die backenartigen Elemente flächig fixiert wird. Durch die außermittige Anordnung der dritten Durchgangsbohrung wird der Verlauf der ersten Durchgangsbohrung bzw. der darin angeordnete Formstift nicht beeinträchtigt. Durch die zweite Durchgangsbohrung wird eine Hebelwirkung zwischen den backenartigen Elementen begünstigt. Vorzugsweise ist der Abstand in axialer Richtung zwischen der dritten Durchgangsbohrung und der zweiten Durchgangsbohrung möglichst groß gewählt, um eine entsprechend große Hebelwirkung zu erzielen.

Nach einem weiteren vorteilhaften Gedanken der Erfindung besteht der Formstift aus einem nichtmetallischen Material. Durch die Verwendung von Formstiften aus nichtmetallischen Materialien können hochreine Glasspritzen, welche keine beziehungsweise lediglich geringe Wolfram- / Metallrückstände aufweisen, hergestellt werden. In derartigen Spritzen können Wirkstoffe teilweise bis zu 3 Jahren gelagert werden, ohne dass der Wirkstoff beeinträchtigt wird.

Vorzugsweise wird der formbare Zustand des Formabschnitts durch Erwärmen realisiert, wobei die Temperatur in einem Bereich zwischen 1000°C und 1200°C, bevorzugt bei etwa 1100°C liegt.

Demzufolge ist es vorteilhaft, wenn die verwendeten Materialien für den Formstift eine Temperaturbeständigkeit bis 1200°C und eine hohe Temperaturwechsel-Beständigkeit (Thermoschockbeständigkeit) aufweisen. Ferner soll das Material geringe Neigung dazu haben, an dem Glasrohling während der Formung zu kleben. Schließlich soll das Material ein wirtschaftlich vertretbares Verhältnis zwischen Standzeit und Anschaffungskosten aufweisen.

Diesen Anforderungen werden technische Keramiken oder keramikartige Materialien gerecht. Demnach besteht der Formstift nach einer besonders bevorzugten Ausführungsform der Erfindung aus einer technischen Keramik oder einem keramikartigen Material. Keramische Werkstoffe sind polykristallin, anorganisch und nichtmetallisch. Sie werden in der Regel bei Raumtemperatur aus einer Rohmasse geformt und erhalten ihre typischen Werkstoffeigenschaften durch einen Sintervorgang, der bei hohen Temperaturen stattfindet. Der Ausdruck technische Keramik ist der Oberbegriff für keramische Werkstoffe und daraus hergestellten Produkten für technische Anwendungen. Technische Keramiken sind weiterhin unterteilt in Silikatkeramiken, Oxidkeramiken und Nichtoxidkeramiken. Die Nichtoxidkeramiken werden weiterhin in carbidische und nitridische Nichtoxidkeramiken unterschieden.

Formstifte basierend auf einer technischen Keramik beziehungsweise keramikartigen Materialien haben zwar höhere Anschaffungskosten als beispielsweise das bisher verwendete Wolfram, jedoch sind mit diesen Materialien wesentlich längere Einsatzzeiten möglich. Im Vergleich zu Platin sind technische Keramiken ebenfalls zu bevorzugen. Zwar sind die Anschaffungskosten vergleichbar, jedoch weisen Keramiken längere Einsatzzeiten als Platin auf. Bei Formstiften basierend auf Keramiken wurden beispielsweise Einsatzzeiten bis zu 24 Stunden festgestellt, wohingegen Formstifte aus Platin Einsatzzeiten von etwa 8 Stunden ermöglichten. Wolframbasierte Formstifte weisen Einsatzzeiten von ca. 1 Stunde auf.

Besonders bevorzugt besteht der Formstift aus Siliziumnitrid (Si₃N₄) oder glasartigen Kohlenstoff. Weitere bevorzugte Materialen sind Zirkoniumoxid und Zirkonium verstärktes Aluminiumoxid (ZTA). Siliziumnitrid ist eine Nichtoxidkeramik und weist eine hohe Bruchzähigkeit und einen niedrigen Wärmeausdehnungskloeffizienten auf. Es zeigte eine Dauergebrauchstemperatur von 1300°C und eine vergleichsweise sehr gute Temperaturwechsel-Belastbarkeit. Glasartiger Kohlenstoff oder Glaskohlenstoff zeigt eine besonders geringe Neigung an dem Glasrohling während der Formung zu kleben, jedoch liegt die Dauergebrauchstemperatur lediglich bei 600°C. Allerdings kann diese Dauergebrauchstemperatur durch Verwendung einer Schutzgasatmosphäre auf über 2000°C erhöht werden.

Die Werkstoffe Siliziumnitrid und glasartiger Kohlenstoff zeichnen sich durch ihre hohe Beständigkeit gegenüber thermischen Wechselwirkungen aus und sind deshalb für den Einsatz als Formstift besonders bevorzugt. Da Siliziumnitrid einen höheren Elastizitätsmodul als glasartiger Kohlenstoff aufweist, können Formstifte aus Siliziumnitrid weniger ausgelenkt werden. Dies hat eine höhere Formtreue der Exzentrizität des Konuskanals zur Folge. Allerdings ist auch eine sehr genaue Positionierung des Formstifts während des Prozesses notwendig, um zu vermeiden, dass der Formstift bricht.

Nach einem bevorzugten Gedanken der Erfindung weist der Formstift einen kegelstumpfförmigen Endbereich und einen zylindrischen Längsbereich mit einem kreisförmigen Querschnitt auf. Durch einen kegelstumpfförmigen Endbereich werden Kanten an dem Formstift vermieden, welche Narben Falten usw. in dem Konuskanal verursachen können vermieden. Ferner wird ein erhöhter Abrieb an den Kanten des Formstifts durch den kegelstumpfförmigen Endbereich vermieden. Vorzugsweise weist der Formstift in dem Längsbereich einen konstanten ersten Durchmesser auf. Offensichtlich wird durch diesen ersten Durchmesser der Innendurchmesser des Kanals vorgegeben. Dieser erste Durchmesser liegt bevorzugt in einem Bereich zwischen 0,7 mm und 1,3 mm und besonders bevorzugt bei etwa 1 mm.

Der Durchmesser des Formstifts muss auf die vorgegebenen Anforderungen an den Glasbehälter angepasst werden. Da bei SIN Spritzen die Kanüle in dem Kanal befestigt bzw. verklebt wird, ist im Vergleich zu Luer-Konus Spritzen bei diesen SIN Spritzen oft ein Kanal geringerem Durchmesser erforderlich. Zu geringe Durchmesser bei Formstiften haben den Nachteil, dass diese schwer zu befestigen sind bzw. schnell brechen. Demnach weist der Formstift gemäß einer weiteren bevorzugten Ausführungsform einen kegelstumpfförmigen Endbereich, einen zylindrischen abgesetzten Bereich und einen zylindrischen Längsbereich auf. Der abgesetzte Bereich und der Längsbereich haben dabei einen kreisförmigen Querschnitt.

Vorzugsweise weist der Längsbereich einen ersten Durchmesser auf, welcher größer ist als der zweite Durchmesser des abgesetzten Bereichs. Die Befestigung des Formstifts kann somit an dem Längsbereich erfolgen, welcher den größeren Durchmesser hat und somit stabiler ist. Gleichzeitig kann ein Kanal mit kleinem Durchmesser durch den abgesetzten Bereich geformt werden. Der abgesetzte Bereich kann sich über die ganze Länge des Kanals erstrecken, wodurch ein Kanal mit einem konstanten Durchmesser geformt wird. Es wäre aber auch denkbar, dass der abgesetzte Bereich sich nur über einen Abschnitt der Kanallänge erstreckt. Demnach würde ein Kanal mit zwei unterschiedlichen Durchmessern ausgeformt.

Im Bereich der SIN Spritzen ist es vorteilhaft wenn der Kanal in einen distalen Abschnitt mit einem größeren Innendurchmesser aufweist. In diesem distalen Bereich wird die Nadel eingesetzt und befestigt bzw. verklebt. Der Übergangsbereich zu dem Abschnitt mit dem kleineren Durchmesser kann als Anlagefläche für die Nadel dienen. Demnach ist es möglich Spritzen mit einer konstanten effektiven Nadellänge herzustellen. Ferner wäre es auch denkbar, dass der Formstift mehrere abgesetzte Bereiche mit unterschiedlichen Durchmessern aufweist. Bevorzugt liegt der erste Durchmesser in einem Bereich zwischen 0,7 und 1,3 mm besonders bevorzugt bei etwa 1 mm. Der zweite Durchmesser liegt bevorzugt in einem Bereich zwischen 0,45 mm und 0,9 mm und besonders bevorzugt bei etwa 0,7 mm. Vorzugsweise weist der Formstift einen Übergangsbereich zwischen dem Längsbereich und dem abgesetzten Bereich auf. Bevorzugt verjüngt sich der Durchmesser des Formstifts in diesem Übergangsbereich kontinuierlich.

Nach einem bevorzugten Gedanken der Erfindung ist der hohlzylindrische Glasrohling an einer Haltevorrichtung derart angeordnet, dass dieser rotierbar ist, wobei die Rotationsachse eine axiale Mittelachse des hohlzylindrischen Glasrohlings ist. Durch die Rotation des Glasrohlings während der Verformung wird eine gleichmäßige Verformung des Glasrohlings gewährleistet.

Vorzugsweise umfasst das zweite Formwerkzeug zwei voneinander beabstandete Formrollen. Die Formrollen sind dabei in einer ersten Position durch einen ersten Abstand beabstandet, wobei zumindest der Formabschnitt des hohlzylindrischen Glasrohlings zwischen die Formrollen verlagerbar ist, wenn die Formrollen sich in der ersten Position befinden.

Vorzugsweise sind die Formrollen zu einer zweiten Position verlagerbar, in welcher diese durch einen zweiten Abstand beabstandet sind, welcher kleiner ist als der erste Abstand. In der zweiten Position ist dabei durch die Formrollen eine Verformungskraft auf den Formabschnitt des hohlzylindrischen Glasrohlings beaufschlagbar, wodurch eine äußere Formgebung des Formabschnitts realisierbar ist. Eine innere Formgebung des Formabschnitts ist durch den Formstift des ersten Formwerkzeugs realisierbar.

Nach einem weiteren vorteilhaften Gedanken der Erfindung umfasst die Vorrichtung eine Kühleinrichtung, durch welche der Formstift kühlbar ist. Durch eine entsprechende Kühlung des Formstifts kann die Einsatzzeit für diesen erhöht werden.

Die Aufgabe der Erfindung wird in einem weiteren Aspekt auch gelöst durch ein Verfahren zur Herstellung eines hochreine innere Oberflächen aufweisenden medizinischen Glasbehälters, umfassend folgende Schritte:
a. Bereitstellen eines sich entlang einer axialen Richtung (X) erstreckenden hohlzylindrischen Glasrohlings mit zumindest einem offenen Ende , wobei der Glasrohling ausgehend von dem offenen Ende einen sich in axialer Richtung (X) erstreckenden formbaren Formabschnitt aufweist, der sich in einem formbaren Zustand befindet;
b. Bereitstellen eines ersten Formwerkzeugs, welches einen Formstift aufweist, wobei der Formstift in einer Fixiereinrichtung des ersten Formwerkzeugs fixiert ist;
c. Bereitstellen eines zweiten Formwerkzeugs, durch welches zumindest der Formabschnitt des hohlzylindrischen Glasrohlings verformbar ist;
d. Einführen des Formstifts durch das offene Ende des hohlzylindrischen Glasrohlings in dessen Formabschnitt;
e. Verformen des Formabschnitts durch das zweite Formwerkzeug derart, dass eine innere Oberfläche des Formabschnitts an dem Formstift anliegt, wodurch der Formabschnitt einen Kanal ausbildet.

Das Verfahren zeichnet sich weiterhin dadurch aus, dass der Formstift in einer Fixiereinrichtung des ersten Formwerkzeugs fixiert wird, wobei die Fixiereinrichtung zumindest zwei backenartige Elemente aufweist, welche an den Formstift flächig anpressbar sind, wodurch der Formstift kraftschlüssig fixiert wird. Vorzugsweise sind dabei die backenartigen Elemente der Fixiereinrichtung einstückig ausgebildet.

Vorzugsweise wird bei dem Verfahren eine Fixiereinrichtung verwendet, welche sich entlang einer axialen Richtung (X') erstreckt und einen ersten Abschnitt, einen als Kreiszylinder ausgebildeten zweiten Abschnitt und einen als Kreiszylinder ausgebildeten dritten Abschnitt umfasst. Bevorzugt weist dabei die Fixiereinrichtung eine erste Durchgangsbohrung auf, welche sich entlang einer axialen Mittelachse der Fixiereinrichtung erstreckt und in welcher der Formstift zumindest abschnittsweise anordenbar ist. Vorteilhaft ist der Formstift derart anordenbar ist, dass dieser über den ersten Abschnitt hinausragt.

Vorzugsweise weist der zweiten Abschnitt der verwendeten Fixiereinrichtung eine erste und eine zweite Schlitzung auf, wobei die backenartigen Elemente durch die erste und die zweite Schlitzung beabstandet sind. Dabei ist bevorzugt die erste Schlitzung in einer ersten Ebene angeordnet, welche durch einen ersten Vektor in axialer Richtung (X') und einen zweiten Vektor in radialer Richtung (Y') aufgespannt wird. Ferner ist es vorteilhaft, wenn die zweite Schlitzung in einer zweiten Ebene angeordnet ist, welche durch den zweiten Vektor und einen dritten Vektor aufgespannt wird, wobei der dritte Vektor einen Winkel α mit der ersten Ebene einschließt. Dieser Winkel α liegt vorzugsweise in einem Bereich zwischen 45° und 125° und besonders bevorzugt bei 90°. Ferner weisen die erste und die zweite Schlitzung eine gemeinsame Schnittgerade auf, welche sich entlang der radialer Richtung (Y') erstreckt.

Nach einem weiteren Gedanken der Erfindung weist der der zweiten Abschnitt der in dem Verfahren verwendeten Fixiereinrichtung entlang der axialen Richtung (X') eine erste Länge auf, wobei die erste Schlitzung entlang der axialen Richtung (X') eine zweite Länge aufweist, welche kleiner als die erste Länge ist.

Vorzugsweise weist die verwendete Fixiereinrichtung in dem zweiten Abschnitt eine zweite Durchgangsbohrung auf, welche sich in der radialen Richtung (Y') erstreckt. Bevorzugt ist die zweite Durchgangsbohrung zu der erste Schlitzung offen und in axialer Richtung zwischen dem ersten Abschnitt der Fixiereinrichtung und der ersten Schlitzung angeordnet.

Der formbare Zustand des Formabschnitts wird vorteilhafterweise durch Erwärmen realisiert, wobei die Temperatur in einem Bereich zwischen 1000°C und 1200°C, bevorzugt bei etwa 1100°C liegt.

Vorzugsweise besteht der verwendete Formstift aus einer technischen Keramik oder einem keramikartigen Material. Besonders bevorzugt besteht der Formstift aus Siliziumnitrid (Si₃N₄) oder glasartigen Kohlenstoff.

Nach einem weiteren bevorzugten Gedanken der Erfindung wird in dem Verfahren ein Formstift verwendet, welcher einen kegelstumpfförmigen Endbereich und einen zylindrischen Längsbereich mit einem kreisförmigen Querschnitt aufweist. Dabei weist der Formstift in dem Längsbereich einen konstanten ersten Durchmesser auf. Bevorzugt liegt der erste Durchmesser in einem Bereich zwischen 0,7 mm und 1,3 mm bevorzugt bei etwa 1 mm.

Nach einem weiteren bevorzugten Gedanken der Erfindung wird in dem Verfahren ein Formstift verwendet, der einen kegelstumpfförmigen Endbereich, einen zylindrischen abgesetzten Bereich und einen zylindrischen Längsbereich aufweist. Dabei weisen der abgesetzte Bereich und der Längsbereich einen kreisförmigen Querschnitt auf. Der Längsbereich weist vorzugsweise einen ersten Durchmesser auf, welcher größer ist als der zweite Durchmesser des abgesetzten Bereichs. Bevorzugt liegt der erste Durchmesser in einem Bereich zwischen 0,7 mm und 1,3 mm besonders bevorzugt bei etwa 1 mm. Der zweite Durchmesser liegt bevorzugt in einem Bereich zwischen 0,45 mm und 0,9 mm und besonders bevorzugt bei etwa 0,7 mm. Vorzugsweise weist der Formstift einen Übergangsbereich zwischen dem Längsbereich und dem abgesetzten Bereich auf. Vorteilhafterweise verjüngt sich der Durchmesser des Formstifts in diesem Übergangsbereich kontinuierlich.

Nach einem vorteilhaften Gedanken der Erfindung der hohlzylindrische Glasrohling an einer Haltevorrichtung angeordnet und wird bei der Verformung rotiert. Die Rotationsachse ist dabei eine axiale Mittelachse des hohlzylindrischen Glasrohlings.

Vorteilhafterweise umfasst das zweite Formwerkzeug zwei voneinander beabstandete Formrollen, wobei die Formrollen in einer ersten Position durch einen ersten Abstand beabstandet sind. Wenn sich die Formrollen sich in der ersten Position befinden, wird vorzugsweise zumindest der Formabschnitt des hohlzylindrischen Glasrohlings zwischen die Formrollen verlagert.

Nach einem weiteren vorteilhaften Gedanken der Erfindung werden die Formrollen zu einer zweiten Position verlagert, in welcher diese durch einen zweiten Abstand beabstandet sind, welcher kleiner ist als der erste Abstand. In dieser zweiten Position wird durch die Formrollen eine Verformungskraft auf den Formabschnitt des hohlzylindrischen Glasrohlings beaufschlagt. Dadurch ist eine äußere Formgebung des Formabschnitts realisierbar. Eine innere Formgebung des Formabschnitts wird vorteilhaft durch den Formstift des ersten Formwerkzeugs realisiert.

Vorzugsweise wird der Formstift durch eine Kühleinrichtung gekühlt.

Weitere Vorteile, Ziele und Eigenschaften der vorliegenden Erfindung werden anhand nachfolgender Beschreibung der anliegenden Figuren erläutert. Gleichartige Komponenten können in den verschiedenen Ausführungsformen gleiche Bezugszeichen aufweisen.

In den Figuren zeigen:
- Fig. 1: eine schematische Anordnung einer Vorrichtung zur Herstellung medizinischen Glasbehälters;
- Fig. 2: eine schematische Anordnung einer Vorrichtung zur Herstellung medizinischen Glasbehälters;
- Fig. 3: eine isometrischen Ansicht eines Formstifts;
- Fig. 4: eine isometrischen Ansicht eines Formstifts gemäß einer weiteren Ausführungsform;
- Fig. 5: eine isometrischen Ansicht einer aus dem Stand der Technik bekannten Fixiereinrichtung;
- Fig. 6: eine Schnittdarstellung einer aus dem Stand der Technik bekannten Fixiereinrichtung;
- Fig. 7: eine isometrischen Ansicht einer aus dem Stand der Technik bekannten Fixiereinrichtung mit einem darin angeordneten Formstift;
- Fig. 8: eine Schnittdarstellung einer aus dem Stand der Technik bekannten Fixiereinrichtung mit einem darin angeordneten Formstift;
- Fig. 9: eine isometrischen Ansicht einer Fixiereinrichtung gemäß einer ersten Ausführungsform;
- Fig. 10: eine Schnittdarstellung einer Fixiereinrichtung gemäß einer ersten Ausführungsform;
- Fig. 11: eine isometrischen Ansicht einer Fixiereinrichtung gemäß einer ersten Ausführungsform mit einem darin angeordneten Formstift;
- Fig. 12: eine Schnittdarstellung einer Fixiereinrichtung gemäß einer ersten Ausführungsform mit einem darin angeordneten Formstift;
- Fig. 13: eine isometrischen Ansicht einer Fixiereinrichtung gemäß einer weiteren Ausführungsform;
- Fig. 14: eine Schnittdarstellung einer Fixiereinrichtung gemäß einer weiteren Ausführungsform;
- Fig. 15: eine isometrischen Ansicht einer Fixiereinrichtung gemäß einer weiteren Ausführungsform mit einem darin angeordneten Formstift;
- Fig. 16: eine Schnittdarstellung einer Fixiereinrichtung gemäß einer weiteren Ausführungsform mit einem darin angeordneten Formstift;
- Fig. 17: eine Schnittdarstellung einer Fixiereinrichtung;
- Fig. 18, 18a: eine Schnittdarstellung einer aus einem Glasrohling gefertigten Spritze;
- Fig. 19a: eine Mikroskop-Aufnahme eines neuen Formstifts aus Wolfram;
- Fig. 19b: eine Mikroskop-Aufnahme eines Formstifts aus Wolfram nach einer Einsatzzeit von 1 Stunde;
- Fig. 19c: eine Mikroskop-Aufnahme eines neuen Formstifts aus Siliziumnitrid;
- Fig. 19d: eine Mikroskop-Aufnahme eines Formstifts aus Siliziumnitrid nach einer Einsatzzeit von 2,5 Stunden.

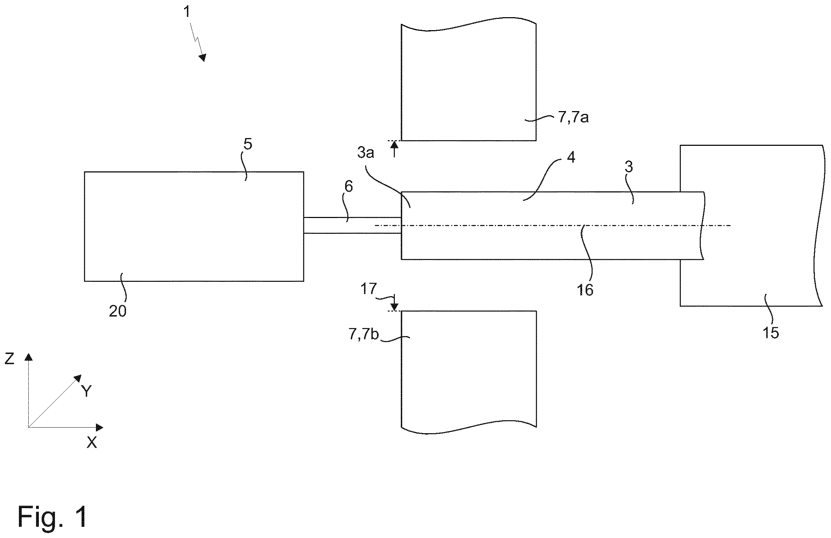

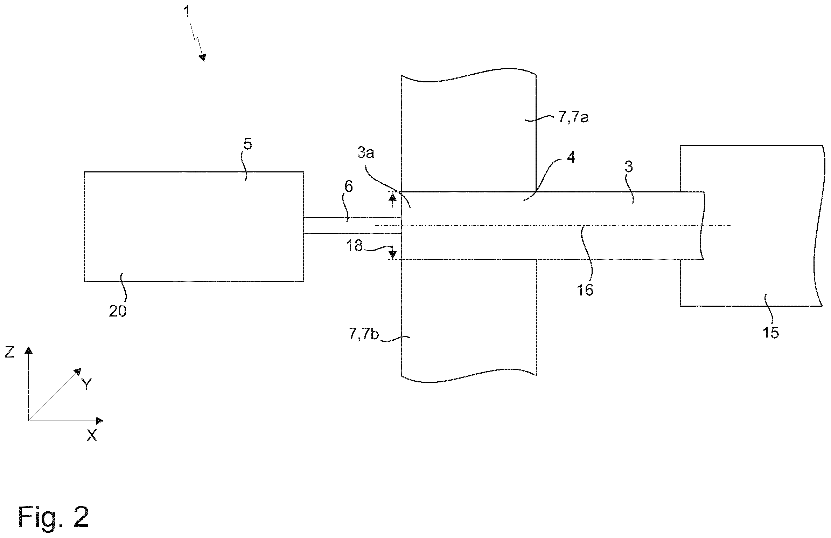

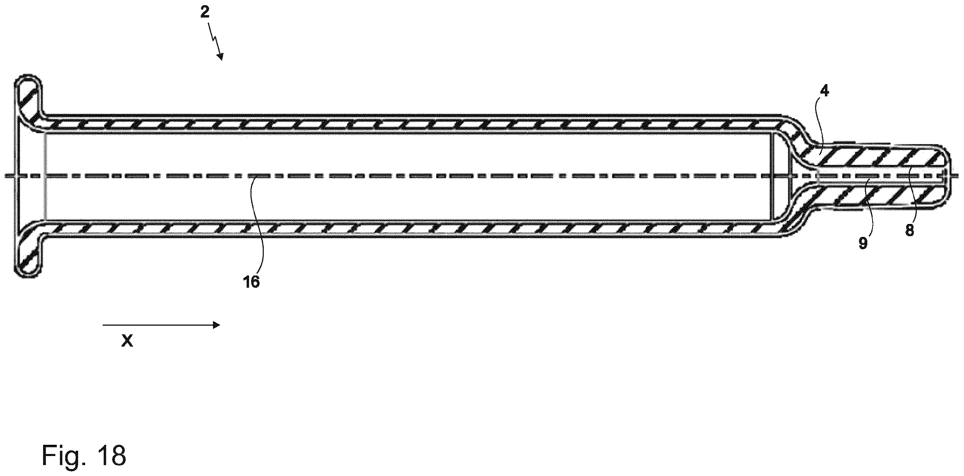

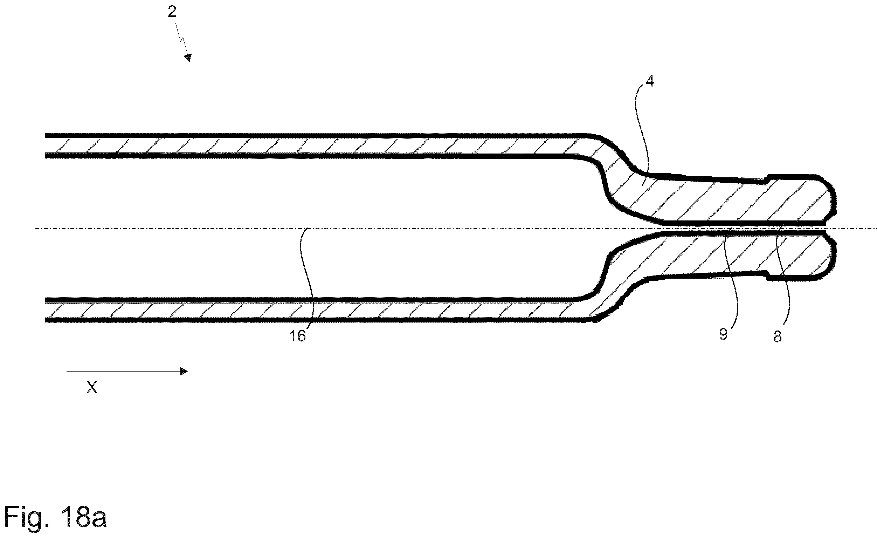

Die Figuren 1 und 2 zeigen eine schematische Anordnung einer Vorrichtung (1) zur Herstellung eines hochreine innere Oberflächen aufweisenden medizinischen Glasbehälters (2). Ein solcher Glasbehälter (2) ist beispielsweis in den Figuren 18 und 18a gezeigt und wird aus einem sich entlang einer axialen Richtung (X) erstreckenden hohlzylindrischen Glasrohling (3) mit zumindest einem offenen Ende (3a) hergestellt. Dieser Glasrohling (3) weist ausgehend von dem offenen Ende (3a) einen sich in axialer Richtung (X) erstreckenden Formabschnitt (4) auf, der sich in einem formbaren Zustand befindet. Der formbare Zustand wird in der Regel durch Erwärmen des Glases realisiert, wobei die Temperatur in einem Bereich zwischen 1000°C und 1200°C, bevorzugt bei etwa 1100°C liegt. Der hohlzylindrische Glasrohling (3) ist an einer Haltevorrichtung (15) derart angeordnet ist, dass dieser rotierbar ist, wobei die Rotationsachse eine axiale Mittelachse (16) des hohlzylindrischen Glasrohlings (3) ist. Durch eine Rotation des Glasrohlings wird eine gleichmäßige Verformung dessen gewährleistet.

Die Vorrichtung (1) umfasst weiterhin ein erstes Formwerkzeug (5), welches einen Formstift (6) aufweist. Dieser Formstift (6) besteht vorzugsweise aus einem nichtmetallischen Material bevorzugt aus einer technischen Keramik oder einem keramikartigen Material und besonders bevorzugt aus Siliziumnitrid (Si₃N₄) oder glasartigen Kohlenstoff. Der Formstift (6) ist durch eine Fixiereinrichtung (20) des ersten Formwerkzeugs (5) fixiert und durch das offene Ende (3a) des hohlzylindrischen Glasrohlings (3) in dessen Formabschnitt (4) entlang der axialen Richtung (X) verlagerbar. Die Fixiereinrichtung (20) weist zumindest zwei backenartige Elemente (21, 22) auf, welche an den Formstift (6) flächig anpressbar sind, wodurch der Formstift (6) kraftschlüssig fixiert wird. Dazu weist das erste Formwerkzeug (5) eine Translationseinrichtung auf, welche die Fixiereinrichtung (20) bzw. den Formstift (6) entlang der axialen Richtung (X) verlagert.

Ferner umfasst die Vorrichtung (1) ein zweites Formwerkzeug (7), durch welches zumindest der Formabschnitt (4) des hohlzylindrischen Glasrohlings (3) verformbar ist. Der Formabschnitt (4) ist durch das zweite Formwerkzeug (7) derart verformbar, dass eine innere Oberfläche (8) des Formabschnitts (4) an dem Formstift (6) anliegt, wodurch der Formabschnitt (4) einen Kanal (9) ausbildet. Das zweite Formwerkzeug (7) umfasst dabei zwei voneinander beabstandete Formrollen (7a, 7b). In der in Figur 1 gezeigten Konfiguration befinden sich die Formrollen (7a, 7b) in einer ersten Position, in welcher sie durch einen ersten Abstand (17) beabstandet sind. Befinden sich die Formrollen (7a, 7b) in dieser ersten Position ist zumindest der Formabschnitt (4) des hohlzylindrischen Glasrohlings (3) zwischen die Formrollen (7a, 7b) verlagerbar. Dies kann beispielsweise über eine entsprechende Transportvorrichtung erfolgen, welche Glasrohlinge der Vorrichtung (1) zuführt und nach der Bearbeitung dem zu dem nächsten Fertigungsschritt transportiert.

In der in Figur 2 gezeigten Konfiguration befinden sich die Formrollen (7a, 7b) in einer zweiten Position. In dieser zweiten Position sind die Formrollen (7a, 7b) durch einen zweiten Abstand (18) voneinander beabstandet. Dieser zweite Abstand (18) ist kleiner als der erste Abstand (17). Die Formrollen liegen an dem Formabschnitt (4) des Glasrohlings (3) an, wodurch eine Verformungskraft auf den Formabschnitt (4) beaufschlagt wird. Somit wird eine äußere Formgebung des Formabschnitts (4) realisiert. Die innere Formgebung des Formabschnitts (4) beziehungsweise die Formgebung des Kanals (9) wird durch Gegenhalten des Formstifts (6) realisiert. Die Form des Kanals (9) ist dabei von der Form des Formstifts (6) abhängig. Um die Einsatzzeiten des Formstifts zu erhöhen, ist es vorteilhaft, wenn die Vorrichtung (1) eine Kühleinrichtung umfasst, durch welche der Formstift (6) kühlbar ist.

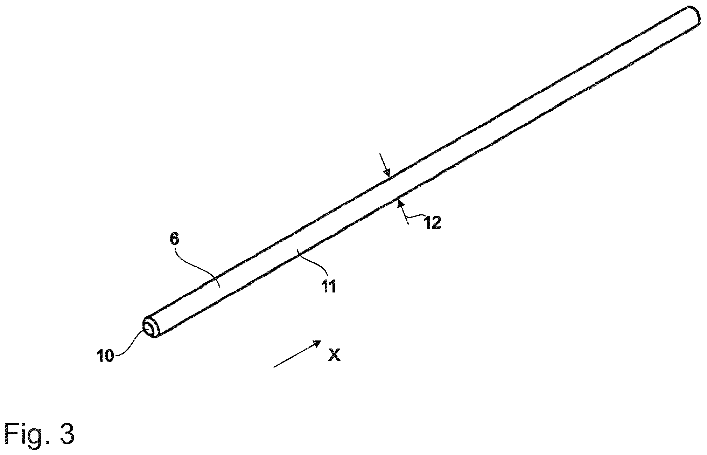

Figur 3 zeigt einen Formstift (6) gemäß einer ersten Ausführungsform. Der Formstift (6) weist einen kegelstumpfförmigen Endbereich (10) und einen zylindrischen Längsbereich (11) auf. Beide Bereiche haben einen kreisförmigen Querschnitt. In dem Längsbereich (11) weist der Formstift (6) einen konstanten ersten Durchmesser (12) auf. Dieser erste Durchmesser (12) kann nach den entsprechenden Anforderungen an den Kanaldurchmesser ausgewählt werden. Bei Verwendung eines solchen Formstifts (6) wird ein Kanal (9) mit konstantem Durchmesser geformt.

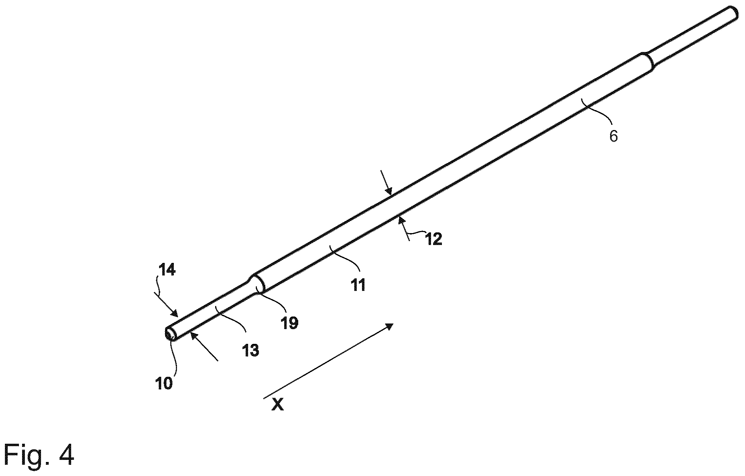

In Figur 4 ist eine weitere Ausführungsform eines Formstifts (6) dargestellt. In dieser Ausführungsform weist der Formstift (6) einen kegelstumpfförmigen Endbereich (10), einen zylindrischen abgesetzten Bereich (13) und einen zylindrischen Längsbereich (11) auf. Die Bereiche haben dabei einen kreisförmigen Querschnitt. Der Längsbereich (11) weist weiterhin einen ersten Durchmesser (12) auf, welcher größer ist als der zweite Durchmesser (14) des abgesetzten Bereichs. Die Befestigung des Formstifts (6) kann somit an seinem Längsbereich (11) erfolgen und gleichzeitig kann ein Kanal (9) mit kleinerem Durchmesser durch den abgesetzten Bereich (13) geformt werden. Der abgesetzte Bereich (13) kann sich über die ganze Länge des Kanals (9) erstrecken, wodurch ein Kanal (9) mit einem konstanten Durchmesser geformt würde. Es wäre aber auch denkbar, dass der abgesetzte Bereich (13) sich nur über einen Abschnitt der Kanallänge erstreckt. Demnach würde ein Kanal (9) mit zwei unterschiedlichen Durchmessern ausgeformt. Der Formstift (6) weist überdies einen Übergangsbereich (19) zwischen dem Längsbereich (11) und dem abgesetzten Bereich (13) auf. Bevorzugt verjüngt sich der Durchmesser des Formstifts (6) in diesem Übergangsbereich (19) kontinuierlich.

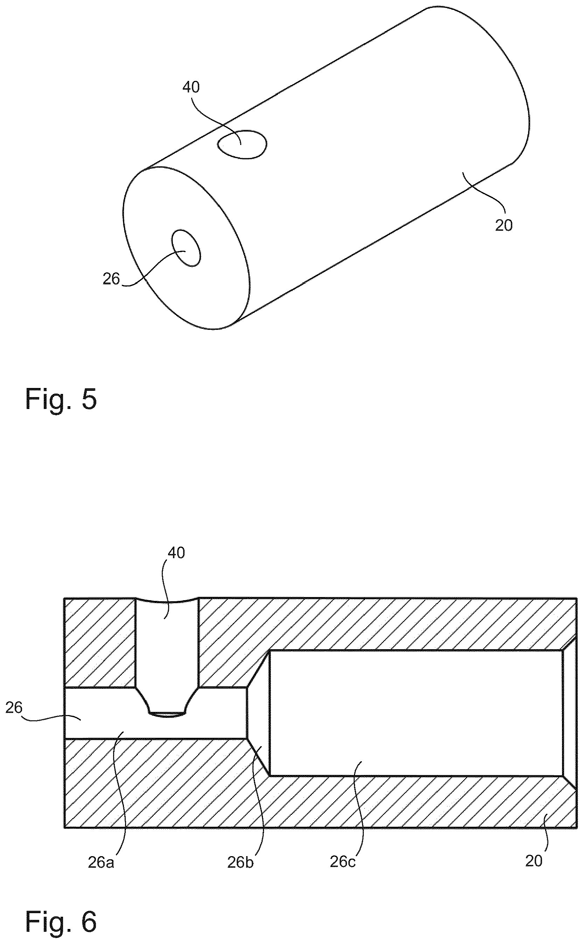

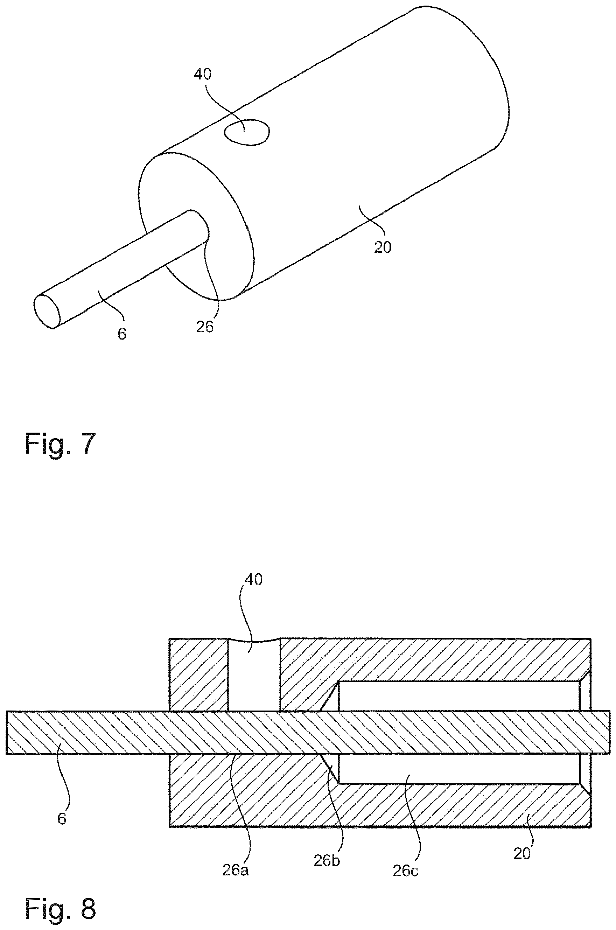

Die Figuren 5 bis 8 zeigen eine aus dem Stand der Technik bekannte Fixiereinrichtung (20). Figur 5 zeigt eine isometrische Ansicht einer aus dem Stand der Technik bekannten Fixiereinrichtung (20), Figur 6 zeigt eine Schnittdarstellung der Fixiereinrichtung (20), Figur 7 zeigt eine isometrische Ansicht der Fixiereinrichtung (20) mit einem darin angeordneten Formstift (6) und Figur 8 zeigt eine Schnittdarstellung der Fixiereinrichtung (20) mit einem darin angeordneten Formstift (6).

Die zylindrische Fixiereinrichtung (20) weist eine Durchgangsbohrung (26) auf, in welcher der Formstift (6) angeordnet wird. Diese Durchgangsbohrung (26) weist wiederum einen ersten Bereich (26a) auf, in welchem der Innendurchmesser der Durchgangsbohrung (26) in etwa dem Außendurchmesser des Formstifts (6) entspricht, beziehungsweise geringfügig größer ist. In diesen ersten Bereich (26a) mündet eine Bohrung (40) in welcher eine Fixierschraube angeordnet werden kann. Durch eine solche Fixierschraube wird der Formstift (6) punktuell fixiert. Werden nun Formstifte (6) mit hoher Sprödigkeit verwendet kann eine solche punktuelle Belastung zum Bruch des Formstifts (6) führen. Ferner weist die Durchgangsbohrung (26) einen zweiten Bereich (26c) auf, in welchem der Innendurchmesser der Durchgangsbohrung (26) erheblich größer ist als der Außendurchmesser des Formstifts (6). In diesem Bereich kann ein Werkzeug zum Wechseln des Formstifts (6) eingeführt werden. Der erste Bereich (26a) ist mit dem zweiten Bereich (26c) über ein Übergangsbereich (26b) verbunden. In diesen Übergangsbereich nimmt der Innendurchmesser zweiten Bereich (26c) kontinuierlich zu dem Innendurchmesser des ersten Bereichs (26a) ab.

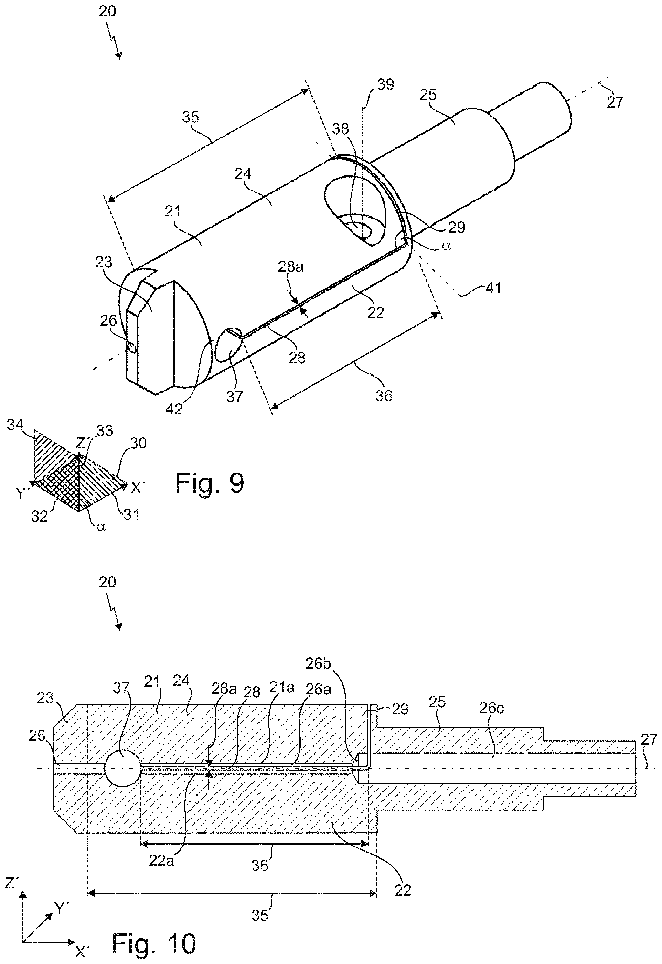

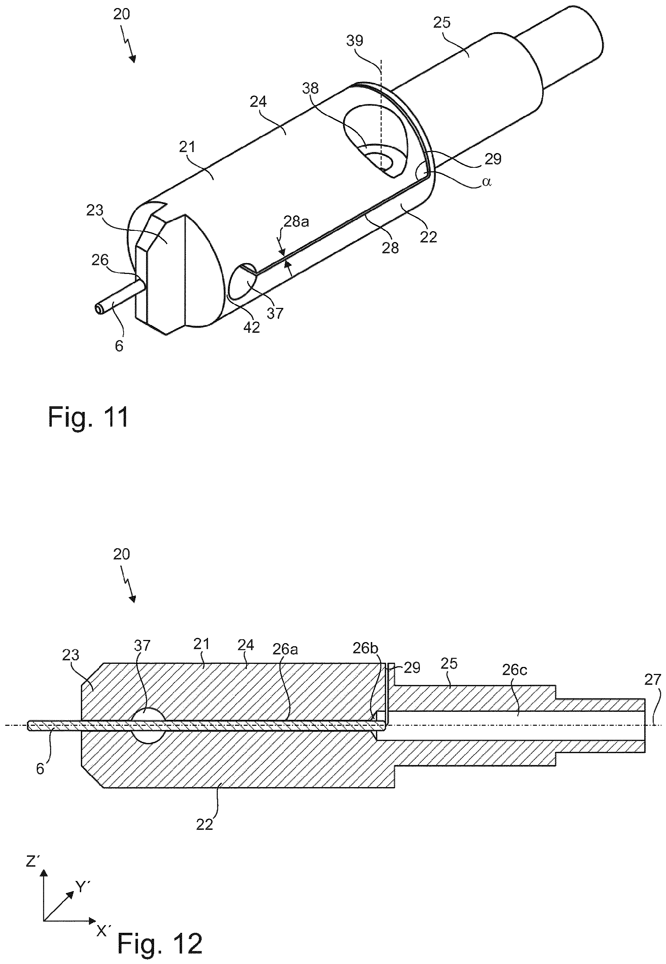

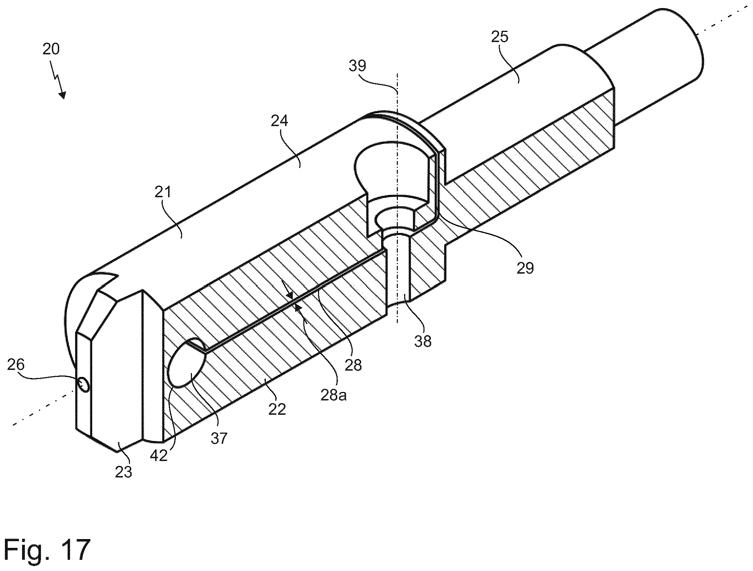

Die Figuren 9 bis 11 zeigen eine Fixiereinrichtung (20) gemäß einer ersten Ausführungsform mit zwei backenartigen Elemente (21, 22), welche an den Formstift (6) flächig anpressbar sind, wodurch der Formstift (6) kraftschlüssig fixiert wird, wobei die backenartigen Elemente (21, 22) der Fixiereinrichtung (20) einstückig ausgebildet sind. Dabei zeigt Figur 9 eine isometrischen Ansicht der Fixiereinrichtung (20), Figur 10 zeigt eine Schnittdarstellung der Fixiereinrichtung (20), Figur 11 zeigt eine isometrische Ansicht der Fixiereinrichtung (20) mit einem darin angeordneten Formstift (6) und Figur 12 zeigt eine Schnittdarstellung der Fixiereinrichtung (20) mit einem darin angeordneten Formstift (6). In Figur 17 ist weiterhin eine isometrische Ansicht der Fixiereinrichtung (20) mit einem außermittigen Schnitt dargestellt.

Die Fixiereinrichtung (20) erstreckt sich entlang einer axialen Richtung (X') und umfasst dabei einen ersten Abschnitt (23), einen als Kreiszylinder ausgebildeten zweiten Abschnitt (24) und einen als Kreiszylinder ausgebildeten dritten Abschnitt (25). Der erste Abschnitt (23) hat eine Quaderform mit Öffnung der Durchgangsbohrung hin abgeschrägten Seitenflächen. Die Fixiereinrichtung (20) weist eine erste Durchgangsbohrung (26) auf, welche sich entlang einer axialen Mittelachse (27) der Fixiereinrichtung (20) erstreckt und in welcher der Formstift (6) zumindest abschnittsweise anordenbar ist, wobei der Formstift (6) derart anordenbar ist, dass dieser über den ersten Abschnitt (23) hinausragt. Dieser hinausragende Abschnitt wird bei der Verformung des Formabschnitts (4) des Glasrohlings (3) durch dessen offenes Ende (3a) in den Formabschnitt (4) verlagert. Der Formabschnitt (4) wird dann durch das zweite Formwerkzeug (7, 7a, 7b) verformt so dass eine innere Oberfläche (8) des Formabschnitts (4) an dem in dem Formabschnitt (4) befindlichem Abschnitt des Formstifts (6) anliegt, wodurch der Formabschnitt (4) des Glasrohlings (3) einen Kanal (9) ausbildet.

Der zweite Abschnitt (24) weist eine erste Länge (35) entlang der axialen Richtung (X') auf. Die backenartigen Elemente (21, 22) sind durch eine erste (28) und eine zweite Schlitzung (29) in dem zweiten Abschnitt voneinander beabstandet. Die erste Schlitzung (28) ist in einer ersten Ebene (30) angeordnet, welche durch einen ersten Vektor (31) in axialer Richtung (X') und einen zweiten Vektor (32) in radialer Richtung (Y') aufgespannt wird. Die zweite Schlitzung (29) ist in einer zweiten Ebene (34) angeordnet, welche durch den zweiten Vektor (32) und einen dritten Vektor (33) aufgespannt wird. Der dritte Vektor (33) schließt einen Winkel α mit der ersten Ebene (30) ein. In diesem Ausführungsbeispiel beträgt der Winkel α 90°. Die erste (28) und die zweite Schlitzung (29) weisen eine gemeinsame Schnittgerade (41) auf, welche sich entlang der radialer Richtung (Y') erstreckt. Die erste Schlitzung (28) hat entlang der axialen Richtung (X') eine zweite Länge (36), welche kleiner als die erste Länge (35) des zweiten Abschnitts ist (24).

Die Fixiereinrichtung (20) weist in dem zweiten Abschnitt (24) eine zweite Durchgangsbohrung (37) auf, welche sich in der radialen Richtung (Y') erstreckt, wobei die zweite Durchgangsbohrung (37) zu der ersten Schlitzung (28) offen ist und in axialer Richtung zwischen dem ersten Abschnitt (23) der Fixiereinrichtung (20) und der ersten Schlitzung (28) angeordnet ist. Der zweite Abschnitt (24) weist weiterhin einen Verbindungsbereich (42) auf, in dem die beiden backenartigen Elemente (21, 22) miteinander verbunden sind.

Ferner weist der zweite Abschnitt (24) eine dritte Durchgangsbohrung (38) auf, deren Mittelachse (39) senkrecht auf der ersten Ebene (30) steht und hinsichtlich der axialen Mittelachse (27) der Fixiereinrichtung (20) radial außermittig angeordnet ist. In der dritten Durchgangsbohrung (38) ist ein Befestigungsmittel anbringbar, durch welches eine Schlitzweite (28a) der ersten Schlitzung (28) reduzierbar ist, wodurch die backenartige Elemente (21, 22) an dem Formstift (6) anpressbar sind. Ein solches Befestigungsmittel könnte beispielsweise eine Spannschraube sein, durch welche eine Kraft auf die beiden backenartigen Elemente (21, 22) beaufschlagt wird, so dass diese zusammengedrückt werden, wodurch der Formstift (6) durch die backenartigen Elemente (21, 22) flächig fixiert wird. Die Durchgangsbohrung weist darüber hinaus eine Senkung auf, in welcher eine solche Spannschraube anordenbar ist. Durch die außermittige Anordnung der dritten Durchgangsbohrung (38) wird der Verlauf der ersten Durchgangsbohrung (26) bzw. der darin angeordnete Formstift (6) nicht beeinträchtigt. Diese außermittige Anordnung der dritten Durchgangsbohrung (38) ist in Figur 17 zu erkennen.

Durch die zweite Durchgangsbohrung (37) wird eine Hebelwirkung zwischen den backenartigen Elementen (21, 22) begünstigt. Vorzugsweise ist der Abstand in axialer Richtung (X') zwischen der dritten Durchgangsbohrung (38) und der zweiten Durchgangsbohrung (37) möglichst groß gewählt, um eine entsprechend große Hebelwirkung zu erzielen.

Die erste Durchgangsbohrung (26) weist einen ersten Bereich (26a) auf, in welchem die Durchgangsbohrung (26) in den backenartigen Elementen (21, 22) ausgebildet ist. In diesem ersten Bereich (26a) ist demnach die Durchgangsbohrung (26) durch die Anlageflächen (21a, 22a) der backenartigen Elemente (21, 22) definiert. Diese Anlageflächen (21a, 22a) sind an den Formstift (6) anpressbar, wodurch dieser flächig fixiert wird. Ferner weist die Durchgangsbohrung (26) einen zweiten Bereich (26c) auf, in welchem der Innendurchmesser der Durchgangsbohrung (26) erheblich größer ist als der Außendurchmesser des Formstifts (6). In diesem Bereich kann ein Werkzeug zum Wechseln des Formstifts (6) eingeführt werden. Der erste Bereich (26a) ist mit dem zweiten Bereich (26c) über ein Übergangsbereich (26b) verbunden. In diesen Übergangsbereich nimmt der Innendurchmesser zweiten Bereich (26c) kontinuierlich zu dem Innendurchmesser des ersten Bereichs (26a) ab.

Die Figuren 9 bis 11 zeigen eine Fixiereinrichtung (20) gemäß einer ersten Ausführungsform mit zwei backenartigen Elemente (21, 22), welche an den Formstift (6) flächig anpressbar sind, wodurch der Formstift (6) kraftschlüssig fixiert wird, wobei die backenartigen Elemente (21, 22) der Fixiereinrichtung (20) einstückig ausgebildet sind. Dabei zeigt Figur 9 eine isometrischen Ansicht der Fixiereinrichtung (20), Figur 10 zeigt eine Schnittdarstellung der Fixiereinrichtung (20), Figur 11 zeigt eine isometrische Ansicht der Fixiereinrichtung (20) mit einem darin angeordneten Formstift (6) und Figur 12 zeigt eine Schnittdarstellung der Fixiereinrichtung (20) mit einem darin angeordneten Formstift (6).

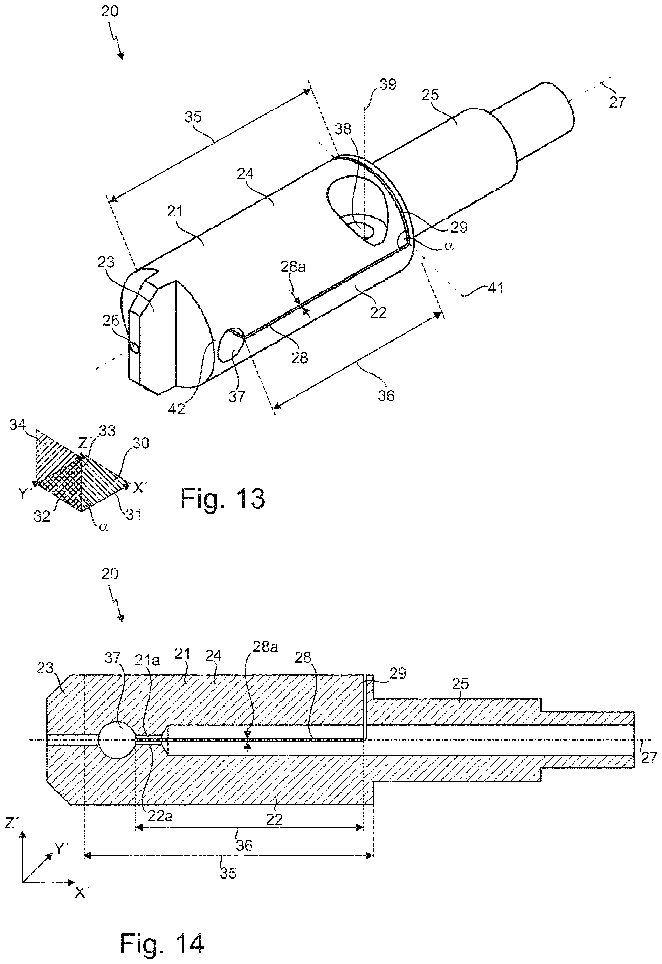

Die Figuren 13 bis 16 zeigen eine Fixiereinrichtung (20) gemäß einer weiteren Ausführungsform mit zwei backenartigen Elemente (21, 22), welche an den Formstift (6) flächig anpressbar sind, wodurch der Formstift (6) kraftschlüssig fixiert wird, wobei die backenartigen Elemente (21, 22) der Fixiereinrichtung (20) einstückig ausgebildet sind. Im Folgenden werden nur die Unterschiede zu der ersten Ausführungsform angeführt. Hinsichtlich gleicher Merkmale wird auf die Beschreibung zu der ersten Ausführungsform verwiesen.

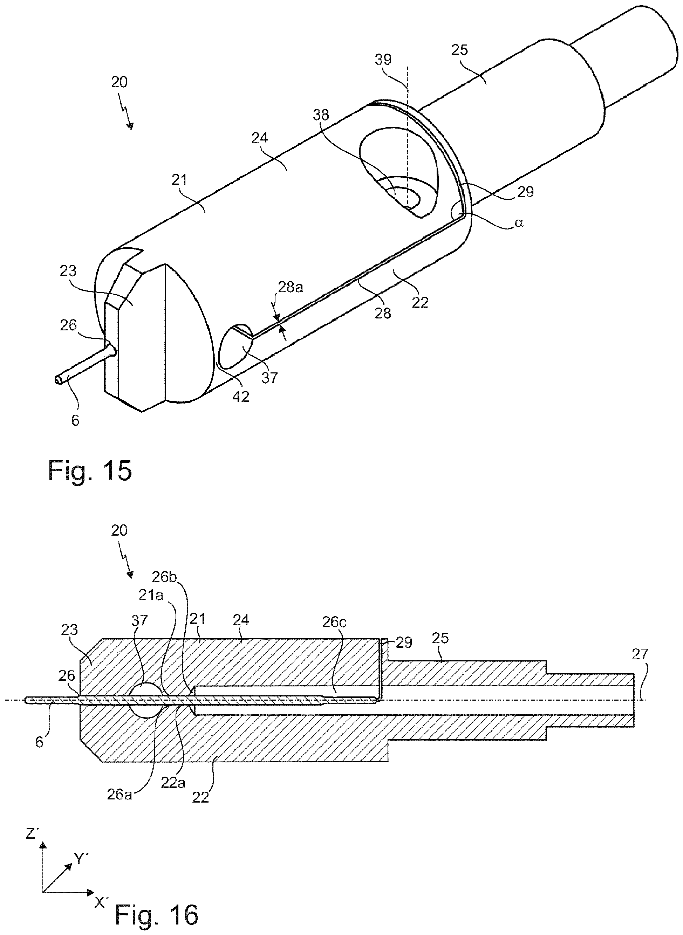

Figur 13 zeigt eine isometrischen Ansicht der Fixiereinrichtung (20) analog zu Figur 9 der ersten Ausführungsform. Figur 14 zeigt eine Schnittdarstellung der Fixiereinrichtung (20) analog zu Figur 10 der ersten Ausführungsform. Figur 15 zeigt eine isometrische Ansicht der Fixiereinrichtung (20) mit einem darin angeordneten Formstift (6) analog zu Figur 11 der ersten Ausführungsform. Figur 16 zeigt eine Schnittdarstellung der Fixiereinrichtung (20) mit einem darin angeordneten Formstift (6) analog zu Figur 12 der ersten Ausführungsform.

Der Unterschied dieser Ausführungsform zu der ersten Ausführungsform ist in den Figuren 14 und 16 gezeigten Schnittdarstellungen zu erkennen und besteht darin, dass der erste Bereich (26a) der Durchgangsbohrung (26) entlang der axialen Richtung (X') eine geringere Länge aufweist. Demnach weisen auch die Anlageflächen (21a, 22a), welche an den Formstift (6) anpressbar sind und durch welche der Formstift (6) flächig fixiert wird, eine geringere Länge auf. Eine solche Fixiereinrichtung (20) ist vorteilhaft bei der Verwendung von Formstiften (6) mit einem abgesetzten Bereich (13), wie beispielsweis in Figur 4 beschrieben. Die Anlageflächen (21a, 22a) würden somit lediglich in dem Längsbereich (11) des Formstifts (6) anliegen welcher einen größeren Durchmesser (12) als der abgesetzte Bereich (13) aufweist.

Figur 18 und Figur 18a zeigen schematisch zwei mögliche Ausführungsformen von medizinischen Glasbehältern (2), welche durch eine erfindungsgemäße Vorrichtung (1) beziehungsweise ein erfindungsgemäßes Verfahren hergestellt wurden. Der medizinische Glasbehälter (2) umfasst ein Endstück, welches aus dem Formabschnitt (4) des hohlzylindrischen Glasrohlings (2) geformt wurde. Bei der in Figur 18a gezeigten Ausführungsform weist das Endstück einen Vorsprung auf, welcher zur Befestigung von Nadelschutzvorrichtungen dienen kann.

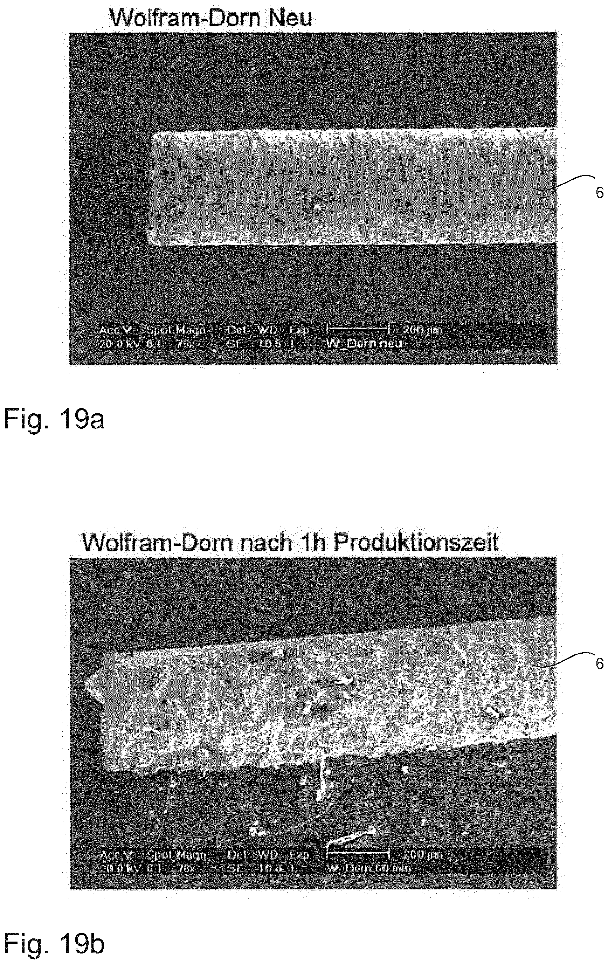

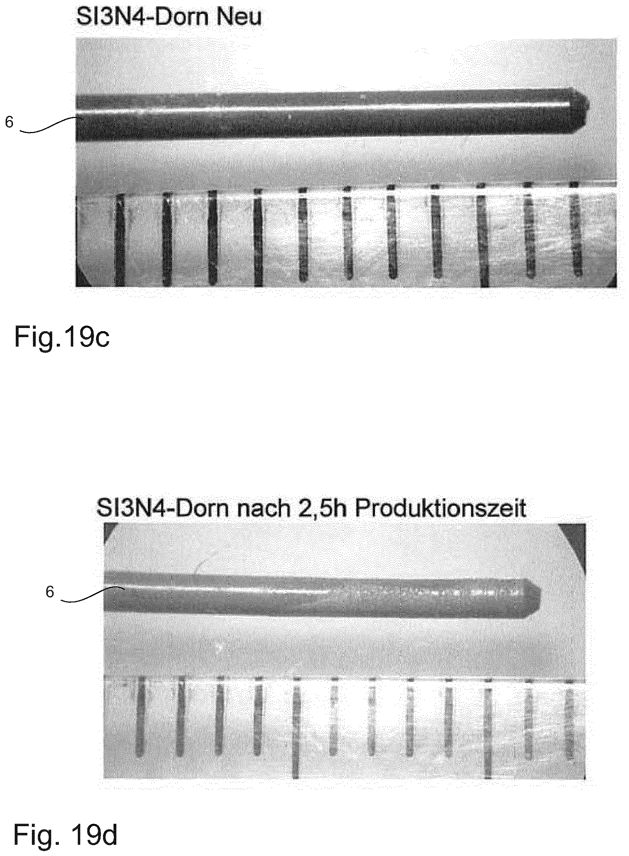

In Figur 19 sind Mikroskop-Aufnahmen von Formstiften (6) dargestellt. In Figur 19a ist ein neuer Formstift (6) aus Wolfram gezeigt. In Figur 19b ist dieser Formstift (6) nach 1 Stunde Produktionszeit dargestellt. Dabei ist deutlich ein gravierender Abrieb an dem Formstift (6) zu erkennen. Figur 19c zeigt einen neuen Formstift (6) aus Siliziumnitrid. In Figur 19d ist dieser Formstift (6) nach 2,5 Stunden Produktionszeit dargestellt. Aus diesen Darstellungen ist ein deutlich geringerer Abrieb des Formstifts aus Siliziumnitrid verglichen mit dem Formstift aus Wolfram zu erkennen.

Analyseuntersuchungen an Glasspritzenkörper, welche aus Siliziumnitrid bzw. glasartigen Kohlenstoff hergestellt wurden, mit Hilfe eines ICP-MS Xs Geräts zeigten einen Wolframgehalt, der 2 Größenordnungen unter dem gemessenen Wolframgehalt von Glasspritzenkörpern liegt, welche mit Wolframformstiften hergestellt wurden.

Sämtliche in den Anmeldungsunterlagen offenbarten Merkmale werden als erfindungswesentlich beansprucht, sofern sie einzeln oder in Kombination gegenüber dem Stand der Technik neu sind.

### Bezugszeichenliste

- 1: Vorrichtung zur Herstellung eines medizinischen Glasbehälters
- 2: medizinischen Glasbehälter
- 3: hohlzylindrischer Glasrohling
- 3a: offenes Ende des hohlzylindrischen Glasrohlings
- 4: Formabschnitt
- 5: erstes Formwerkzeug
- 6: Formstift
- 7: zweites Formwerkzeug
- 7a, 7b: Formrollen
- 8: innere Oberfläche des Formabschnitts
- 9: Kanal
- 10: kegelstumpfförmigen Endbereich des Formstifts
- 11: Längsbereich des Formstifts
- 12: erster Durchmesser
- 13: abgesetzter Bereich des Formstifts
- 14: zweiter Durchmesser
- 15: Haltevorrichtung
- 16: axiale Mittelachse des Glasrohlings
- 17: erster Abstand zwischen den Formrollen
- 18: zweiter Abstand zwischen den Formrollen
- 19: Übergangsbereich
- 20: Fixiereinrichtung
- 21: erstes backenartiges Element
- 21a: Anlagefläche des ersten backenartigen Elements
- 22: zweites backenartiges Element
- 22a: Anlagefläche des zweiten backenartigen Elements
- 23: erster Abschnitt der Fixiereinrichtung
- 24: zweiter Abschnitt der Fixiereinrichtung
- 25: dritter Abschnitt der Fixiereinrichtung
- 26: erste Durchgangsbohrung der Fixiereinrichtung
- 26a: erster Bereich der ersten Durchgangsbohrung der Fixiereinrichtung
- 26b: Übergangsbereich der ersten Durchgangsbohrung der Fixiereinrichtung
- 26c: zweiter Bereich der ersten Durchgangsbohrung der Fixiereinrichtung
- 27: axiale Mittelachse der Fixiereinrichtung
- 28: erste Schlitzung
- 28a: Schlitzweite der ersten Schlitzung
- 29: zweite Schlitzung
- 30: erste Ebene
- 31: erster Vektor
- 32: zweiter Vektor
- 33: dritter Vektor
- 34: zweite Ebene
- 35: erste Länge
- 36: zweite Länge
- 37: zweite Durchgangsbohrung
- 38: dritte Durchgangsbohrung
- 39: Mittelachse der dritten Durchgangsbohrung
- 40: Bohrung
- 41: Schnittgerade
- 42: Verbindungsbereich

## Patentansprüche

1. Vorrichtung (1) zur Herstellung eines hochreinen medizinischen Glasbehälters (2) aus einem sich entlang einer axialen Richtung (X) erstreckenden hohlzylindrischen Glasrohling (3) mit zumindest einem offenen Ende (3a), wobei der Glasrohling (3) ausgehend von dem offenen Ende (3a) einen sich in axialer Richtung (X) erstreckenden Formabschnitt (4) aufweist, der sich in einem formbaren Zustand befindet, wobei die Vorrichtung (1) umfasst:
a. ein erstes Formwerkzeug (5), welches einen Formstift (6) aufweist, wobei der Formstift (6) durch das offene Ende (3a) des hohlzylindrischen Glasrohlings (3) in dessen Formabschnitt (4) entlang der axialen Richtung (X) verlagerbar ist, wobei der Formstift (6) in einer Fixiereinrichtung (20) des ersten Formwerkzeugs (5) fixiert ist;
b. ein zweites Formwerkzeug (7), durch welches zumindest der Formabschnitt (4) des hohlzylindrischen Glasrohlings (3) verformbar ist, wobei der Formabschnitt (4) durch das zweite Formwerkzeug (7) derart verformbar ist, dass eine innere Oberfläche (8) des Formabschnitts (4) an dem Formstift (6) anliegt, wodurch der Formabschnitt (4) einen Kanal (9) ausbildet;
**dadurch gekennzeichnet, dass**
die Fixiereinrichtung (20) zumindest zwei backenartige Elemente (21, 22) aufweist, welche an den Formstift (6) flächig anpressbar sind, wodurch der Formstift (6) kraftschlüssig fixierbar ist.

2. Vorrichtung (1) nach Anspruch 1,
**dadurch gekennzeichnet, dass**
die backenartigen Elemente (21, 22) der Fixiereinrichtung (20) einstückig ausgebildet sind.

3. Vorrichtung (1) nach Anspruch 1 oder 2,
**dadurch gekennzeichnet, dass**
die Fixiereinrichtung (20) sich entlang einer axialen Richtung (X') erstreckt und einen ersten Abschnitt (23), einen als Kreiszylinder ausgebildeten zweiten Abschnitt (24) und einen als Kreiszylinder ausgebildeten dritten Abschnitt (25) umfasst, wobei die Fixiereinrichtung (20) eine erste Durchgangsbohrung (26) aufweist, welche sich entlang einer axialen Mittelachse (27) der Fixiereinrichtung (20) erstreckt und in welcher der Formstift (6) zumindest abschnittsweise anordenbar ist, wobei der Formstift (6) derart anordenbar ist, dass dieser über den ersten Abschnitt (23) hinausragt.

4. Vorrichtung (1) nach Anspruch 3,
**dadurch gekennzeichnet, dass** der zweite Abschnitt (24) eine erste (28) und eine zweite Schlitzung (29) aufweist, wobei die backenartigen Elemente (21, 22) durch die erste (28) und die zweite Schlitzung (29) beabstandet sind, wobei die erste Schlitzung (28) in einer ersten Ebene (30) angeordnet ist, welche durch einen ersten Vektor (31) in axialer Richtung (X') und einen zweiten Vektor (32) in radialer Richtung (Y') aufgespannt wird, wobei die zweite Schlitzung (29) in einer zweiten Ebene (34) angeordnet ist, welche durch den zweiten Vektor (32) und einen dritten Vektor (33) aufgespannt wird, wobei der dritte Vektor (33) einen Winkel α mit der ersten Ebene (30) einschließt, wobei die erste (28) und die zweite Schlitzung (29) eine gemeinsame Schnittgerade (41), welche sich entlang der radialer Richtung (Y') erstreckt, aufweisen.

5. Vorrichtung (1) nach Anspruch 4,
**dadurch gekennzeichnet, dass** der zweite Abschnitt (24) entlang der axialen Richtung (X') eine erste Länge (35) aufweist, wobei die erste Schlitzung (28) entlang der axialen Richtung (X') eine zweite Länge (36) aufweist, welche kleiner als die erste Länge (35) ist.

6. Vorrichtung (1) nach Anspruch 4 oder 5,
**dadurch gekennzeichnet, dass**
die Fixiereinrichtung (20) in dem zweiten Abschnitt (24) eine zweite Durchgangsbohrung (37) aufweist, welche sich in der radialen Richtung (Y') erstreckt, wobei die zweite Durchgangsbohrung (37) zu der ersten Schlitzung (28) offen ist und in axialer Richtung zwischen dem ersten Abschnitt (23) der Fixiereinrichtung (20) und der ersten Schlitzung (28) angeordnet ist.

7. Vorrichtung (1) nach einem der vorhergehenden Ansprüche 1 - 6,
**dadurch gekennzeichnet, dass**
der zweite Abschnitt (24) eine dritte Durchgangsbohrung (38) aufweist, deren Mittelachse (39) senkrecht auf der ersten Ebene (30) steht und hinsichtlich der axialen Mittelachse (27) der Fixiereinrichtung (20) radial außermittig angeordnet ist, wobei in der dritten Durchgangsbohrung (38) ein Befestigungsmittel anbringbar ist, durch welches eine Schlitzweite (28a) der ersten Schlitzung (28) reduzierbar ist, wodurch die backenartige Elemente (21, 22) an dem Formstift (6) anpressbar sind.

8. Vorrichtung (1) nach einem der vorhergehenden Ansprüche,
**dadurch gekennzeichnet, dass**
der Formstift (6) aus einem nichtmetallischen Material besteht.

9. Vorrichtung (1) nach einem der vorhergehenden Ansprüche,
**dadurch gekennzeichnet, dass**
der Formstift (6) aus einer technischen Keramik besteht.

10. Vorrichtung (1) nach einem der vorhergehenden Ansprüche 1-8, **dadurch gekennzeichnet, dass** der Formstift (6) aus Siliziumnitrid (Si₃N₄) oder glasartigem Kohlenstoff besteht.

11. Vorrichtung (1) nach einem der vorhergehenden Ansprüche,
**dadurch gekennzeichnet, dass**
der Formstift (6) einen kegelstumpfförmigen Endbereich (10) und einen zylindrischen Längsbereich (11) mit einem kreisförmigen Querschnitt aufweist, wobei der Formstift (6) in dem Längsbereich (11) einen konstanten ersten Durchmesser (12) aufweist, wobei der erste Durchmesser (12) in einem Bereich zwischen 0,7 mm und 1,3 mm bevorzugt bei 1 mm liegt.

12. Vorrichtung (1) nach einem der vorhergehenden Ansprüche 1-10,
**dadurch gekennzeichnet, dass**
der Formstift (6) einen kegelstumpfförmigen Endbereich (10), einen zylindrischen abgesetzten Bereich (13) und einen zylindrischen Längsbereich (11), wobei der abgesetzte Bereich (13) und der Längsbereich (11) einen kreisförmigen Querschnitt aufweisen, wobei der Längsbereich (11) einen ersten Durchmesser (12) aufweist, weleher größer ist als der zweite Durchmesser (14) des abgesetzten Bereichs, wobei der erste Durchmesser (12) in einem Bereich zwischen 0,7 und 1,3 mm bevorzugt bei 1 mm liegt und der zweite Durchmesser (14) in einem Bereich zwischen 0,45 mm und 0,9 mm bevorzugt bei 0,7 mm liegt.

13. Vorrichtung (1) nach einem der vorhergehenden Ansprüche,
**dadurch gekennzeichnet, dass**
das zweite Formwerkzeug (7) zwei voneinander beabstandete Formrollen (7a, 7b) umfasst, wobei die Formrollen (7a, 7b) in einer ersten Position durch einen ersten Abstand (17) beabstandet sind, wobei zumindest der Formabschnitt (4) des hohlzylindrischen Glasrohlings (3) zwischen die Formrollen (7a, 7b) verlagerbar ist wenn, die Formrollen (7a, 7b) sich in der ersten Position befinden.

14. Vorrichtung (1) nach einem der vorhergehenden Ansprüche,
**dadurch gekennzeichnet, dass**
die Formrollen (7a, 7b) zu einer zweiten Position verlagerbar sind, in welcher diese durch einen zweiten Abstand (18) beabstandet sind, welcher kleiner ist als der erste Abstand (17), wobei in der zweiten Position durch die Formrollen (7a, 7b) eine Verformungskraft auf den Formabschnitt (4) des hohlzylindrischen Glasrohlings (3) beaufschlagbar ist, wodurch eine äußere Formgebung des Formabschnitts (4) realisierbar ist, wobei eine innere Formgebung des Formabschnitts (4) durch den Formstift (6) des ersten Formwerkzeugs (5) realisierbar ist.

15. Verfahren zur Herstellung eines hochreine innere Oberflächen aufweisenden medizinischen Glasbehälters (2), umfassend folgende Schritte:
a. Bereitstellen eines sich entlang einer axialen Richtung (X) erstreckenden hohlzylindrischen Glasrohlings (3) mit zumindest einem offenen Ende (3a), wobei der Glasrohling (3) ausgehend von dem offenen Ende (3a) einen sich in axialer Richtung (X) erstreckenden formbaren Formabschnitt (4) aufweist;
b. Bereitstellen eines ersten Formwerkzeugs (5), welches einen Formstift (6) aufweist, wobei der Formstift (6) in einer Fixiereinrichtung (20) des ersten Formwerkzeugs (5) fixiert ist;
c. Bereitstellen eines zweiten Formwerkzeugs (7), durch welches zumindest der Formabschnitt (4) des hohlzylindrischen Glasrohlings (3) verformbar ist;
d. Einführen des Formstifts (6) durch das offene Ende (3a) des hohlzylindrischen Glasrohlings (3) in dessen Formabschnitt (4);
e. Verformen des Formabschnitts (4) durch das zweite Formwerkzeug (7) derart, dass eine innere Oberfläche (8) des Formabschnitts (4) an dem Formstift (6) anliegt, wodurch der Formabschnitt (4) einen Kanal (9) ausbildet;
**dadurch gekennzeichnet, dass**
die Fixiereinrichtung (20) zumindest zwei backenartige Elemente (21, 22) aufweist, welche an den Formstift (6) flächig anpressbar sind, wodurch der Formstift (6) kraftschlüssig fixiert wird.

## Claims

1. Device (1) for producing a high-purity medical glass container (2) from a hollow cylindrical glass blank (3) extending along an axial direction (X) and having at least one open end (3a), the glass blank (3) having a moulded section (4) extending in the axial direction (X) from the open end (3a), which section is in a mouldable state, the device (1) comprising:
a. a first moulding tool (5) having a moulding pin (6), the moulding pin (6) being able to be moved via the open end (3a) of the hollow cylindrical glass blank (3) in the moulded section (4) thereof along the axial direction (X), the moulding pin (6) being fixed in a fixing unit (20) of the first moulding tool (5);
b. a second moulding tool (7), via which at least the moulded section (4) of the hollow cylindrical glass blank (3) can be deformed, the moulded section (4) being able to be deformed by the second moulding tool (7) in such a way that an inner surface (8) of the moulded section (4) is in contact with the moulding pin (6), whereby the moulded section (4) forms a channel (9),
**characterised in that**
the fixing unit (20) has at least two jaw-type elements (21, 22) which can be pressed extensively on the moulding pin (6), whereby the moulding pin (6) can be force-lockingly fixed.

2. Device (1) according to claim 1,
**characterised in that**
the jaw-type elements (21, 22) of the fixing unit (20) are formed in one piece.

3. Device (1) according to either claim 1 or claim 2,
**characterised in that**
the fixing unit (20) extends along an axial direction (X') and comprises a first section (23), a second section (24) that is formed as a circular cylinder, and a third section (25) that is formed as a circular cylinder, the fixing unit (20) having a first through bore (26) that extends along an axial central axis (27) of the fixing unit (20) and in which the moulding pin (6) can be arranged at least in sections, the moulding pin (6) being able to be arranged so as to protrude beyond the first section (23).

4. Device (1) according to claim 3,
**characterised in that**
the second section (24) has a first (28) and a second slot (29), the jaw-type elements (21, 22) being spaced apart by means of the first (28) and the second slot (29), the first slot (28) being arranged in a first plane (30) that is spanned by a first vector (31) in the axial direction (X') and by a second vector (32) in the radial direction (Y'), the second slot (29) being arranged in a second plane (34) that is spanned by the second vector (32) and by a third vector (33), the third vector (33) and the first plane (30) enclosing an angle α, the first (28) and the second slot (29) having a common intersection line (41) which extends along the radial direction (Y').

5. Device (1) according to claim 4,
**characterised in that**
the second section (24) has a first length (35) along the axial direction (X'), the first slot (28) having a second length (36) along the axial direction (X') that is smaller than the first length (35).

6. Device (1) according to either claim 4 or claim 5,
**characterised in that**
the fixing unit (20) has a second through bore (37) in the second section (24), which bore extends in the radial direction (Y'), the second through bore (37) being open towards the first slot (28) and being arranged between the first section (23) of the fixing unit (20) and the first slot (28) in the axial direction.

7. Device (1) according to any of the preceding claims 1-6,
**characterised in that**
the second section (24) has a third through bore (38), the central axis (39) of which is perpendicular to the first plane (30) and is radially off-centre with respect to the axial central axis (27) of the fixing unit (20), it being possible for a fastening means to be fastened in the third through bore (38), which fastening means can reduce a slot width (28a) of the first slot (28), whereby the jaw-type elements (21, 22) can be pressed on the moulding pin (6).

8. Device (1) according to any of the preceding claims,
**characterised in that**
the moulding pin (6) consists of a non-metallic material.

9. Device (1) according to any of the preceding claims,
**characterised in that**
the moulding pin (6) consists of a technical ceramics.

10. Device (1) according to any of the preceding claims 1-8,
**characterised in that**
the moulding pin (6) consists of a silicon nitride (Si₃N₄) or glass-like carbon.

11. Device (1) according to any of the preceding claims,
**characterised in that**
the moulding pin (6) has a frustoconical end region (10) and a cylindrical longitudinal region (11) having a circular cross section, the moulding pin (6) having a constant first diameter (12) in the longitudinal region (11), the first diameter (12) being in a range of between 0.7 mm and 1.3 mm, and preferably being 1 mm.

12. Device (1) according to any of the preceding claims 1-10,
**characterised in that**
the moulding pin (6) has a frustoconical end region (10), a cylindrical reduced region (13), and a cylindrical longitudinal region (11), the reduced region (13) and the longitudinal region (11) having a circular cross section, the longitudinal region (11) having a first diameter (12) which is larger than the second diameter (14) of the reduced region, the first diameter (12) being in a range of between 0.7 and 1.3 mm, and preferably being 1 mm, and the second diameter (14) being in a range of between 0.45 mm and 0.9 mm, and preferably being 0.7 mm.

13. Device (1) according to any of the preceding claims,
**characterised in that**
the second moulding tool (7) has two mutually spaced shaping rollers (7a, 7b), the shaping rollers (7a, 7b) being spaced apart by a first spacing (17) when in a first position, it being possible for at least the moulded section (4) of the hollow cylindrical glass blank (3) to be moved between the shaping rollers (7a, 7b) when the shaping rollers (7a, 7b) are in the first position.

14. Device (1) according to any of the preceding claims,
**characterised in that**
the shaping rollers (7a, 7b) can be moved into a second position in which said rollers are spaced apart by a second spacing (18) that is smaller than the first spacing (17), the shaping rollers (7a, 7b) being able to apply a deformation force to the moulded section (4) of the hollow cylindrical glass blank (3) when in the second position, whereby an outer shaping of the moulded section (4) can be achieved, it being possible for an inner shaping of the moulded section (4) to be achieved by means of the moulding pin (6) of the first moulding tool (5).

15. Method for producing a medical glass container (2) having high-purity inner surfaces, said method comprising the following steps:
a. providing a hollow cylindrical glass blank (3) extending along an axial direction (X) and having at least one open end (3a), the glass blank (3) having a mouldable moulded section (4) extending in the axial direction (X) from the open end (3a);
b. providing a first moulding tool (5) having a moulding pin (6), the moulding pin (6) being fixed in a fixing unit (20) of the first moulding tool (5);
c. providing a second moulding tool (7), via which at least the moulded section (4) of the hollow cylindrical glass blank (3) can be deformed;
d. inserting the moulding pin (6) via the open end (3a) of the hollow cylindrical glass blank (3) in the moulded section thereof (4);
e. deforming the moulded section (4) by the second moulding tool (7) in such a way that an inner surface (8) of the moulded section (4) is in contact with the moulding pin (6), whereby the moulded section (4) forms a channel (9);
**characterised in that**
the fixing unit (20) has at least two jaw-type elements (21, 22) which can be pressed extensively on the moulding pin (6), whereby the moulding pin (6) is force-lockingly fixed.

## Revendications

1. Dispositif (1) de fabrication d'un récipient médical (2) en verre de haute pureté à partir d'une ébauche de verre cylindrique creuse (3) s'étendant le long d'une direction axiale (X) et comportant au moins une extrémité ouverte (3a), l'ébauche de verre (3) présentant en partant de l'extrémité ouverte (3a) une section de moulage (4) s'étendant en direction axiale (X), qui se trouve dans un état moulable, le dispositif (1) comportant:
a. un premier outil de moulage (5), lequel présente une broche de moulage (6), la broche de moulage (6) étant déplaçable le long de la direction axiale (X) par l'extrémité ouverte (3a) de l'ébauche de verre cylindrique creuse (3) dans sa section de moulage (4), la broche de moulage (6) étant fixée dans un dispositif de fixation (20) du premier outil de moulage (5);
b. un second outil de moulage (7) par lequel au moins la section de moulage (4) de l'ébauche de verre cylindrique creuse (3) est déformable, la section de moulage (4) étant déformable par le second outil de moulage (7) de telle sorte qu'une surface interne (8) de la section de moulage (4) s'applique sur la broche de moulage (6), ce par quoi la section de moulage (4) forme un canal (9);
**caractérisé par le fait que**
le dispositif de fixation (20) présente au moins deux éléments de type mâchoire (21, 22), lesquels sont aptes à être pressés en surface sur la broche de moulage (6), ce par quoi la broche de moulage (6) est apte à être fixée à force.

2. Dispositif (1) selon la revendication 1,
**caractérisé par le fait que**
les éléments en forme de mâchoire (21, 22) du dispositif de fixation (20) sont réalisés d'un seul tenant.

3. Dispositif (1) selon l'une des revendications 1 ou 2,
**caractérisé par le fait que**
le dispositif de fixation (20) s'étend le long d'une direction axiale (X') et comporte une première section (23), une deuxième section (24) réalisée en tant que cylindre à base circulaire et une troisième section (25) réalisée en tant que cylindre à base circulaire, le dispositif de fixation (20) présentant un premier alésage traversant (26), lequel s'étend le long d'un axe central axial (27) du dispositif de fixation (20) et dans lequel la broche de moulage (6) est apte à être disposée au moins dans certaines régions, la broche de moulage (6) étant apte à être disposée de telle sorte que celle-ci fait saillie au-delà de la première section (23).

4. Dispositif (1) selon la revendication 3,
**caractérisé par le fait que**
la deuxième section (24) présente une première (28) et une seconde fente (29), les éléments en forme de mâchoire (21, 22) étant espacés par la première (28) et la seconde fente (29), la première fente (28) étant disposée dans un premier plan (30), lequel est défini par un premier vecteur (31) en direction axiale (X') et un deuxième vecteur (32) en direction radiale (Y'), la seconde fente (29) étant disposée dans un second plan (34), lequel est défini par le deuxième vecteur (32) et un troisième vecteur (33), le troisième vecteur (33) formant avec le premier plan (30) un angle a, la première (28) et la seconde fente (29) présentant une ligne d'intersection commune (41), laquelle s'étend le long de la direction radiale (Y').

5. Dispositif (1) selon la revendication 4,
**caractérisé par le fait que**
la deuxième section (24) présente une première longueur (35) le long de la direction axiale (X'), la première fente (28) présentant le long de la direction axiale (X') une seconde longueur (36), laquelle est plus petite que la première longueur (35).

6. Dispositif (1) selon l'une des revendications 4 ou 5,
**caractérisé par le fait que**
le dispositif de fixation (20) présente dans la deuxième section (24) un second alésage traversant (37), lequel s'étend dans la direction radiale (Y'), le second alésage traversant (37) étant ouvert vers la première fente (28) et étant disposé en direction axiale entre la première section (23) du dispositif de fixation (20) et la première fente (28).

7. Dispositif (1) selon l'une des revendications 1 à 6,
**caractérisé par le fait que**
la deuxième section (24) présente un troisième alésage traversant (38), dont l'axe central (39) est perpendiculaire au premier plan (30) et est disposé radialement excentré par rapport à l'axe central axial (27) du dispositif de fixation (20), un moyen de fixation étant apte à être fixé dans le troisième alésage traversant (38), moyen par lequel une largeur de fente (28a) de la première fente (28) est apte à être réduite, ce par quoi les éléments en forme de mâchoire (21, 22) sont aptes à être pressés sur la broche de moulage (6).

8. Dispositif (1) selon l'une des revendications précédentes,
**caractérisé par le fait que**
la broche de moulage (6) est constituée d'un matériau non métallique.

9. Dispositif (1) selon l'une des revendications précédentes,
**caractérisé par le fait que**
la broche de moulage (6) est constituée d'une céramique technique.

10. Dispositif (1) selon l'une des revendications 1 à 8,
**caractérisé par le fait que**
la broche de moulage (6) est constituée de nitrure de silicium (Si₃N₄) ou de carbone vitreux.

11. Dispositif (1) selon l'une des revendications précédentes,
**caractérisé par le fait que**
la broche de moulage (6) présente une zone d'extrémité tronconique (10) et une zone longitudinale cylindrique (11) comportant une section transversale circulaire, la broche de moulage (6) présentant un premier diamètre constant (12) dans la zone longitudinale (11), le premier diamètre (12) se situant dans une plage entre 0,7 mm à 1,3 mm, de préférence étant de 1 mm.

12. Dispositif (1) selon l'une des revendications 1 à 10,
**caractérisé par le fait que**
la broche de moulage (6) présente une zone d'extrémité tronconique (10), une zone réduite cylindrique (13) et une zone longitudinale cylindrique (11), la zone réduite (13) et la zone longitudinale (11) présentant une section transversale circulaire, la zone longitudinale (11) présentant un premier diamètre (12), lequel est plus grand que le second diamètre (14) de la zone réduite, le premier diamètre (12) se situant dans une plage entre 0,7 et 1,3 mm, de préférence étant de 1 mm, et le second diamètre (14) se situant dans une plage entre 0,45 mm et 0,9 mm, de préférence étant de 0,7 mm.

13. Dispositif (1) selon l'une des revendications précédentes,
**caractérisé par le fait que**
le second outil de moulage (7) comporte deux rouleaux de formage espacés l'un de l'autre (7a, 7b), les rouleaux de formage (7a, 7b) étant espacés par une première distance (17) dans une première position, au moins la section de moulage (4) de l'ébauche de verre cylindrique creuse (3) étant déplaçable entre les rouleaux de formage (7a, 7b) lorsque les rouleaux de formage (7a, 7b) se trouvent dans la première position.

14. Dispositif (1) selon l'une des revendications précédentes,
**caractérisé par le fait que**
les rouleaux de formage (7a, 7b) sont déplaçables vers une seconde position dans laquelle ceux-ci sont espacés par une seconde distance (18), laquelle est plus petite que la première distance (17), une force de déformation étant apte à être appliquée sur la section de moulage (4) de l'ébauche de verre cylindrique creuse (3) dans la seconde position par les rouleaux de formage (7a, 7b), ce par quoi un façonnage externe de la section de moulage (4) est réalisable, un façonnage interne de la section de moulage (4) étant réalisable par la broche de moulage (6) du premier outil de moulage (5).

15. Procédé de fabr0ication d'un récipient médical en verre (2) présentant des surfaces internes de haute pureté, comportant les étapes suivantes:
a. se procurer une ébauche de verre cylindrique creuse (3) s'étendant le long d'une direction axiale (X) et comportant au moins une extrémité ouverte (3a), l'ébauche de verre (3) présentant en partant de l'extrémité ouverte (3a) une section de moulage (4) moulable s'étendant en direction axiale (X);
b. se procurer un premier outil de moulage (5), lequel présente une broche de moulage (6), la broche de moulage (6) étant fixée dans un dispositif de fixation (20) du premier outil de moulage (5);
c. se procurer un second outil de moulage (7), par lequel au moins la section de moulage (4) de l'ébauche de verre cylindrique creuse (3) est déformable;
d. introduire la broche de moulage (6) par l'extrémité ouverte (3a) de l'ébauche de verre cylindrique creuse (3) dans sa section de moulage (4);
e. déformer la section de moulage (4) par le second outil de moulage (7) de telle sorte qu'une surface interne (8) de la section de moulage (4) s'applique sur la broche de moulage (6), ce par quoi la section de moulage (4) forme un canal (9);
**caractérisé par le fait que**
le dispositif de fixation (20) présente au moins deux éléments en forme de mâchoire (21, 22), lesquels sont aptes à être pressés en surface sur la broche de moulage (6), ce par quoi la broche de moulage (6) est fixée à force.
